(19)

Europäisches Patentamt
European Patent Office
Office européen des brevets

(11) **EP 4 454 586 A1**

(12) **EUROPEAN PATENT APPLICATION**

(43) Date of publication:
**30.10.2024 Bulletin 2024/44**

(21) Application number: **23315110.9**

(22) Date of filing: **26.04.2023**

(51) International Patent Classification (IPC):
**A61B 34/10** (2016.01)   **A61B 90/00** (2016.01)
**A61B 34/00** (2016.01)   **A61N 7/00** (2006.01)
**A61N 7/02** (2006.01)   A61B 34/20 (2016.01)

(52) Cooperative Patent Classification (CPC):
**A61B 90/37; A61B 34/10; A61B 34/25; A61N 7/00;**
**A61N 7/02;** A61B 34/20; A61B 2034/104;
A61B 2034/105; A61B 2034/107; A61B 2034/2074;
A61B 2090/365; A61B 2090/378

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Theraclion SA**
**92240 Malakoff (FR)**

(72) Inventors:
• **Grisey, Anthony**
**78210 Saint Cyr l'Ecole (FR)**
• **Gerold, Björn**
**78000 Versailles (FR)**
• **Anquez, Jérémie**
**75013 Paris (FR)**
• **Barnat, Nesrine**
**95870 Bezons (FR)**

(74) Representative: **Hepp Wenger Ryffel AG**
**Friedtalweg 5**
**9500 Wil (CH)**

(54) **SYSTEM FOR GATHERING TREATMENT DATA AND/OR GENERATING A TREATMENT PLAN FOR TREATING A PATIENT WITH HITU AND METHOD FOR DEFINING TREATMENT PARAMETERS**

(57)    The invention relates to a system (1) for gathering treatment data and/or generating a treatment plan for treating a patient with HITU. The system comprises a computer device (10) which can be brought in operative connection with a memory (12). The computer device (10) is adapted to receive, via an interface (11), first imaging data of a body region and second imaging data (20) of the body region. The system (1) is preferably further adapted to create a first reconstructed model (22, 23, 23') of the body region before a treatment and a second reconstructed model (22, 23, 23") of the body region after treatment has been conducted based on the first and second imaging data (20), and/or preferably to receive a first reconstructed model (22, 23, 23') of the body region before a treatment and a second reconstructed model (22, 23, 23'') of the body region after treatment forming first and second imaging data (20). The system (1), preferably the computer device (10), is further adapted to, based on the first and the second reconstructed models (22, 23, 23', 23"), generate an outcome metric for at least two segments (24', 24") of the reconstructed first and second model (22, 23, 23', 23'') and save the outcome metric in the memory (12).

Fig. 1a

EP 4 454 586 A1

**Description**

The invention relates to a system for gathering treatment data and/or generating a treatment plan for treating a patient with HITU (High-Intensity Therapeutic Ultrasound, which includes High-Intensity Focused Ultrasound), a method for defining treatment parameter, a method of treating a patient, a computer program product according to the preamble of the independent claims.

It is generally known to use data, for example of prior treatments, to plan or conduct a treatment.

EP 1 907 065 discloses a method of adapting a treatment plan. The method includes preparing a treatment plan for treating a patient condition which includes acquiring an internal reference image of the patient. Furthermore, three-dimensional internal images of the patient in a treatment position are generated.

US 6,968,224 discloses a surgical instrument navigation system which comprises an ultrasound machine and a computer coupled to the ultrasound machine. A memory is coupled to the computer and includes computer instructions that when executed by the computer cause the computer to generate an icon representing the surgical instrument's trajectory and to overlay the icon on a real-time ultrasound image having an image plane.

US 8,112,292 discloses a method and apparatus to perform a procedure that can include a processor assisted surgical procedure. During the procedure patient space and image space can be registered to allow for tracking of various tracking sensors.

US 2020/113543 discloses a method of diagnosing a condition of bodily tissue using a computer by comparing, using a computer, a 3D tissue model derived from an ultrasound scan of the bodily tissue with at least one 3D tissue model having common tissue with the bodily tissue. Diagnosis of a condition of the bodily tissue is done by comparing the 3D tissue models.

US 7,693,257 discloses a method and apparatus to optimize delivery of radiation treatment.

US 2016/174895 provides systems and methods for monitoring tissue regions and in particular changes in tissue regions over a period of time.

Furthermore, extraction and reconstruction of 3D models of veins and other organs is known in the art and commerciallyavailable for diagnostic purposes.

DE 10 2014 111066 A1 describes ways to capture the position of an ultrasound probe and to perform the 3D model extraction. However, extracted 3D models are not linked to a medical procedure, such as a therapy or positioning a patient or a treatment head. In particular, registration of a current position of a treatment device within a reconstructed volumes or extracted 3D model is complex.

However, the prior art only provides for limited planning and prediction of treatments. In particular, systems and methods known in the art do not provide sufficiently precise control over the performed treatment.

It is therefore an object of the present invention to overcome the drawbacks of the prior art, in particular to provide a systems and methods that allow for more precise treatment planning and conduction.

This and other objects are achieved with the systems and methods according to the characterizing portion of the independent claims.

In some embodiments, imaging data or other data is used to train algorithms, preferably artificial intelligence (AI) algorithms, aiming at optimizing a therapy, preferably a High-Intensity Therapeutic Ultrasound (HITU) treatment, of a structure of interest, preferably a vein.

The present invention may further provide, in some embodiments a way to acquire and register data, to define an outcome or success metric of a treatment based on acquired data, and using artificial intelligence algorithms to optimize the outcome of a therapy. Further disclosed are appropriate interfaces to perform these tasks.

The invention relates to a system for gathering data related to a treatment, in particular imaging data and/or treatment charac-teristics and/or position and/or orientation data related to one or several imaging and/or treatment devices, and/or generating a treatment plan for treating a patient with HITU. The system comprises a computer device which can be brought in operable connection with a memory.

Optionally, the system may comprise a first and a second computer device. In particular, it can be advantageous to perform certain computing steps on separate computer devices. For example, a first computer may be associated with a treatment device and collect data and/or control the treatment device, while a second device is arranged such as to receive and treat data from the first device. In the following, it should be understood that any reference to a computer device may mean either one of the first and second computer device (or any further computer device where more than two are present).

It is also possible that the system comprises three or more computer devices. For example, one server may be used to host the database, another server for registration of imaging date, another server may be used for the computation of outcome metrics, another one for training of models, and so forth.

The computer device is adapted to receive, via an interface, first imaging data of a body region and second imaging data of the body region. The interface may be part of a control unit. The first imaging data may correspond to the body region before a treatment has occurred. The second imaging, data may correspond to substantially the same body region either after treatment has occurred or during a treatment. Thus, comparison of the first and second imaging data may typically allow for determination of certain effects of a particular treatment. During a treatment may in particular mean where a patient is in a lying position (or in Trendelenburg position) and/or a treatment head applies pressure to a tissue to be treated.

Imaging data may refer to an image or one to several sets of images, preferably medical images such as B-mode images, duplex images, Computed Tomography volumes etc., preferably complemented with position and/or orientation data enabling to know the relative position and/or orientation of at least a subset of the images with respect to each other. These position and/or orientation data can for example be generated by a robot or a tracking system, for example an optical tracking system, an electromagnetic tracking system, an accelerometer, a gyroscope, or a combination therein.

Alternatively, the imaging data received by the computer may be processed data based on an image or one to several sets of images, preferably medical images, preferably complemented with position and/or orientation data.

In the following "acquired data" will refer to substantially raw data originating from the imaging device which may be complemented by additional data such as data originating from sensors, for example position and/or orientation sensors. "Prepared model" will refer to processed data which are suitable for an outcome metric computation, and "intermediate data" will refer to at least partially processed data based on "acquired data", in particular at any intermediate step between "acquired data" and "prepared model". In particular, if the outcome metric computation requires spatial consistency between the first and second imaging data, the prepared models related to the same treatment may be spatially consistent with respect to each other. Spatial consistency may be obtained, for example, based on a posteriori registration or because the acquired data were directly captured in the same frame of reference, and/or comprise position and/or orientation data with respect to the patient's anatomy.

In the following "reconstructed model" may .refer to acquired data or data derived from acquired data such as intermediate data or prepared data, possibly complemented with other additional data (e.g. patient data).

In general, models created or used in the methods, devices and systems disclosed herein may be a reconstructed model.

In practice all data encompassed in the terms "acquired data" or "reconstructed model" may be physically stored in the same memory at some stage of the process. Alternatively, "acquired data" or "reconstructed model" may only refer to a logical group of data but, for example, the position data may only be stored in the same memory as the imaging data.

In the present invention, the imaging data received by the computer may be:

- "acquired data" or "intermediate data", in which case processing steps, such as segmentation, registration, volume reconstruction may be performed, preferably by the computer device or one of the computer devices, to enable an outcome metric computation.

- "prepared model", in which case an outcome metric computation can be directly performed.

Thus, preferably, the system is adapted to create a first prepared model of the body region before a treatment and a second prepared model of the body region after a treatment has been conducted. In this case, the first and second prepared models can be based on the first and second imaging data. Additionally or alternatively, the system may be adapted to receive a first prepared model of the body region before a treatment and a second prepared model of the body region after treatment, in which case the first and second prepared models form the first and second imaging data.

Any other combination of acquired data, intermediate data and/or prepared model is possible. For example, it is also possible that one imaging data is a prepared model, and the other imaging data consists in acquired or intermediate data. For example, it may be advantageous to compute a first prepared model or intermediate data based on the acquired data captured before treatment, to display some information regarding the anatomical structure of interest or to serve as input for treatment planning for example. Then, depending on the system architecture, after treatment, the system may receive this first intermediate data or prepared model as first imaging data and may receive the acquired data captured after treatment as second imaging data. Additionally or alternatively, depending on the system architecture, and in particular if the acquired data captured before treatment was stored in the memory of the system before treatment and kept until the second imaging data is received, the first imaging data may for example comprise or consist of acquired data collected before treatment and the second imaging may be acquired data collected after treatment.

First and second imaging data may thus be received substantially simultaneously by the system, during or after treatment; alternatively, the reception of the second imaging data may arise later than the reception of the first imaging data, potentially several days, months or even years later. For example, the first imaging data can be received before treatment and stored until the second imaging data is received.

One set of first imaging data may also serve for several outcome metric computations in combination with different second sets of imaging data. For example, the outcome metric of a treatment can be computed in acute based on first imaging data and second im- - aging data collected shortly after treatment. Then, it can be recomputed after a certain period of time based on the same first imaging data and another set of second imaging data collected during a follow-up visit of a patient for example.

The system is further adapted to generate an outcome metric for at least two segments of the prepared first and second models. The outcome metric is generated based on the first and second prepared model. Preferably, the outcome metric is generated by the computer device. In embodiments that have a first and second computer device, the outcome metric may be generated by either one of the first and second computer device (or any other computer device which may be present). The system is further adapted to save the outcome metrics on the memory.

In some embodiments, the outcome metric is generated based on the first and second prepared model and at least a third prepared model. For example, the third prepared model may relate to a different timepoint compared to the second prepared model so as to provide an outcome metric which takes into account temporal development of a treatment.

In one particularly preferred embodiment, the outcome metric is generated by the second computer whereas the imaging data is received by the first computer.

Here and in the following, an outcome metric shall be understood as a parameter representing a change in characteristics of one or several tissues, preferably a local change directly induced by the treatment. It is preferably quantitative (not only binary for example). For example, an outcome metric can be a change in a vessel diameter before and after a treatment with HITU. An outcome metric can also be a change in tissue depth between the skin of a patient and a vessel before and during treatment. Outcome metrics may be measured in absolute terms, i.e. as an absolute change in a tissue characteristic. The outcome metric may also be measured in relative terms, e.g. as a ratio before and during/after treatment. The outcome metric can be a scalar-valued function (e.g. a change in vessel diameter is a scalar value) or a vector-valued function (e.g. returning the change in vessel diameter and the area of damaged perivenous tissue orthogonally to the main vein axis for each position along the vein). The outcome metric may also comprise or consist of a qualitative or semi-quantitative assessment (e.g. low, medium, high reduction in size) or to a classification of the output (e.g. success, failure, moderate effect). The computation of the outcome metric may also take into account the global result of a treatment and aggregate it with local information. For example, in the context of a HITU treatment of a vein, slices may be assigned to a class "success" when the local shrinkage is greater than a certain threshold (e.g. 50 %) and the vein is globally occluded, and assigned to a class "failure" when the vein is globally open and refluxing and the local shrinkage is below a certain threshold (e.g. 30 %). Some segments of the anatomical structure may not be considered when computing the outcome metric, for example because of an effect which is hard to interpret (e.g., in the context of a HITU treatment of veins, a nominally sufficient shrinkage of a vein in a segment after treatment but which does not occlude the vein (i.e. the vein remained open and reflux occurs) may be not be easy to classify as "success" or "failure"). Additionally or alternatively, the outcome metric may be computed for the whole structure or the whole part of the structure impinged by the treatment and some segments are discarded afterwards during the subsequent analyses. The outcome metric is preferably a function of at least a spatial variable (e.g. the 1D position along a vein). Preferably, if one of.the tissues for which the outcome metric represents a change in characteristics is a vessel, the outcome metric preferably characterizes the local contraction of the vessel (preferably computed based on the diameters before and after treatment) and/or the local occlusion and/or local reflux abolition when applicable.

A segment shall generally be understood as a slice or cross-section of the model. The segments can have a finite thickness and need not be strictly two-dimensional. For example, where a reconstructed model represents a vessel in a patient's body, a segment maybe a two-dimensional cross-section of the model, preferably in a plane perpendicular to the longitudinal axis of the vessel. Alternatively, the segment may also have a finite thickness. Typically, the thickness of the segment is less than 70 mm, preferably less than 10 mm. In general, a segment represents a local part of a global region. A segment, irrespective of its thickness, need not span an entire cross-section of a volume. For example, a segment may represent a sector of a cross-section of a vessel. A segment may be a treatment slice or can be another arbitrary slice of the model.

The thickness of the segment does not necessarily reflect the thickness of the zone in which the effect of the treatment is characterized by the value of the outcome metric. For example, in the context of a HITU treatment comprising pulses delivered in treatment slices located along a vessel, the outcome metric may be the vessel shrinkage (e.g. computed as the difference between the diameter before treatment and the diameter after treatment, divided by the diameter before treatment) at discrete locations along the vessel, but it may be interpreted as characterizing the effect of the treatment along segments of finite thickness (e.g. encompassing several, preferably a fix number of, treatment slices).

A segment may be a portion of one or several anatomical structures of interest on which the outcome metric is computed. For example, if the outcome metric is the shrinkage of a vein (e.g. computed as the difference between the diameter before treatment and the diameter after treatment, divided by the diameter before treatment), a segment could be a localized area positioned along the vein. It could also be a portion of the vein of a certain length (e.g. 5 mm), in which case the diameters before and after treatment can be taken as a function of the spatially-varying diameter of the vein before and after treatment (e.g. the mean, the median, the value in the geometrical center of the vein portion etc.). In this context, a target may also comprise or consist of one or multiple segments. It may also encompass several anatomical structures- (e.g. a vein and the perivenous tissue).

The treatment (in particular, the HITU treatment) of vessels is particularly suited to the computation of an outcome metric. Indeed, the thermal treatment of a vessel (in particular varicose veins) may induce a radial shrinkage of the vessel, for example in the plane orthogonal to its main axis, but the locations of the segments along the vessel are preserved. Thus, the distance between segments (e.g. treatment slices) along the main axis of the vessel may be preserved after treatment. This allows for a direct computation of the vessel shrinkage, for example, by comparing the radius of the vessel before and after treatment at the same location along its main axis.,By contrast, a tumor may shrink in all directions as a result of thermal or radiotherapy treatment, thus requiring a more complex registration process to find a correspondence between the segments before and after treatment, since their relative position changes-with respect to each other.

Along with data enabling outcome metric computation, reconstructed models may also comprise information which is suitable for or especially computed for display. For example, when the body region of interest is a thigh or a vein in the thigh, it can be a mesh of the 3D conformation of the vein or a collection of 3D meshes of the vein and the surrounding structures (other veins, arteries, nerves, skin...), or a collection of diameters indexed by their position along the vein enabling to represent the vein as a cylinder of varying diameter along its main axis.

Preferably, the first and second imaging data are based on or comprise images of a same imaging modality. For example, the first and second imaging data may be both based_ on B-mode imaging data, or both on color Doppler imaging data. Alternatively, the first and second imaging data are based on different imaging modalities.

Particularly preferably, the first and second imaging data are acquired by the same imaging device.

Most preferably, imaging data are of the same type, i.e. "acquired data", or "intermediate data", or "prepared models".

Reconstructed models related to first and second imaging data may, but do not necessarily, contain exactly the same information.

For example, a reconstructed model before treatment may contain more information, preferably information which may have an impact on the treatment. In an example where the reconstructed models are reconstructed models of an incompetent vein before and after a HITU treatment, a representative diameter of the vein or a collection of vein diameters and vein depths under skin indexed by their position along the vein can be captured. They can for example serve as input parameters when trying to understand the influence of some parameters on the outcome metric, for example during a model training.

In another example, the outcome metric computation is based on simpler information acquired at another timepoint. For example, the assumption might be made that the diameter of a vein before treatment is uniform all along the vein. Then, the outcome metric may be computed as the difference between the spatially-varying diameter extracted from the reconstructed model after treatment and a representative diameter acquired before treatment, divided by the representative diameter acquired before treatment. In this case, the first prepared model is a scalar value.

Preferably the prepared models are spatially registered with respect to each other to enable direct comparison of state of the structure before and after treatment.

Registration may be partial or come from a partial information. In an example where the imaging data is a prepared model formed by a collection of vein diameters and their corresponding coordinates along the main longitudinal axis of the vein, and the original images are substantially orthogonal to the vein, complemented with relative position information within each of the two imaging data, it may be enough to register the spatial position of the images serving to generate the two imaging data between the two imaging data along the main longitudinal axis of the vein.

In a preferred embodiment, the position or orientation data are directly captured with respect to the patient anatomy in the body region of interest or comprise position and/or orientation data related to several points substantially fixed with respect to the patient anatomy in the body region of interest. For example, in the context of optical tracking, markers can be placed directly on the patient anatomy (e.g. using one or several disposable electro cardiogram patches or other skin-compatible adhesives) and/or on wearables (e.g, a kneepad) and/or on an external structure (e.g. a handrail or a dedicated stage on which the patient is standing during imaging data acquisitions), and their position and orientation is also captured by the tracking system. These markers may be substantially at the same position with respect to the target of the treatment or another part of the patient's anatomy during the acquisition of the first and second imaging data and they can serve as references and enable direct registration of the reconstructed models.

Alternatively or additionally, anatomical landmarks (e.g. junction between veins, remarkable locations along bones, lymph nodes) can also serve as reference. Typically, the person performing the image acquisition could indicate to the system the position of the landmarks (e.g. by using a dedicated control or by staying still during a certain period of time, e.g. 2 seconds, when reaching a landmark).

Alternatively or additionally, reconstructed models before and after treatment are registered based on the content of the acquired images using manual registration or automatic registration algorithms known in the art. For example, anatomical landmarks (e.g. junctions with tributaries) may be automatically identified or segmented in the reconstructed models and serve as basis for registration. Other information can also be used alone or in combination, such as the position of the sapheno-femoral junction when applicable, or the profile of the distance to the skin along the target.

The first and second reconstructed models are preferably three-dimensional models such 3D meshes, 3D label maps, a list of diameters (and preferably depths under skin) with 1D coordinates along a vein etc.

Reconstructed models may be related to one or several anatomical structures. For example, if the treatment may damage the tissue surrounding a vein, the reconstructed models (preferably at least the second reconstructed model) can comprise a model of the vein (e.g. a list of diameters with 1D coordinates along a vein) and a model of the damaged volume around the vein (e.g. under the form or a collection of outlines with 1D coordinates along the vein). In this example, the first imaging data may differ in content from the second imaging data because it may be hypothesized that the damaged volume around the vein is null before treatment, thus the first imaging data may not contain this information.

Particularly preferably, the first and second reconstructed models or, at least, the part of the reconstructed models serving for outcome metric computation, are of the same nature, i.e. a reconstruction of the same body region under the same format (for example, collection of diameters with 1D coordinates along a vein), even where the data acquired before and after/during treatment are not of the same type. For example, it would be possible to estimate the diameters of a vein along its course before treatment based on B-mode images, and the diameters immediately after treatment based on color Doppler information. For example, this would enable to identify acute thrombosis, since acute thrombi are not always visible in B-mode, and to only segment the portion of the vein lumen which is not occluded. Reconstructed models can also be based on multimodal imaging data and/or group several types of information. For example, computing the diameter of a vein along its longitudinal axis before and after a treatment may not be sufficient to determine the outcome of a treatment. Typically, a vein occlusion is not visible in acute on B-mode images. Thus, preferably at least after treatment, it may be advantageous to complement the information of diameter by color Doppler data to assess occlusion. This can be done by performing a second scan of the vein in color Doppler mode or by acquiring color Doppler information at discrete positions along the vein. Alternatively or additionally, compressibility of the vein may be assessed during the B-mode scan of the vein or during a dedicated scan, either by constantly applying a strong pressure onto the vein or by applying a strong pressure at discrete locations along the vein. As known by a person skilled in the art, these acquisitions can be performed in any suitable order, during the same movement along the vein (e.g. proximal to distal) or during several scans. The results may be stored in one or several files. Usually, the ultrasound scanner will save the color Doppler acquisitions and the B-mode acquisitions in different files. They may still be considered as one set of acquired imaging data. Preferably, the data composing a set of acquired imaging data are registered between each other. Registration within one set of acquired data is usually direct, e.g. if the patient does not move and an optical tracking system is used, but manual registration or automatic registration algorithms can also be used to register the different acquisitions between them if needed.

The reconstructed model may be a voxelized reconstructed volume. For example, an algorithm may loop over at least a portion of, preferably all, pixels of one, several or all acquired images and assign them to a nearest voxel of a reconstructed volume. Then the value of each voxel within the reconstructed volume can be taken as the mean value of the values of each assigned pixel.

Linear interpolation can be used to assign a value to the voxels of the reconstructed volume to which no pixel was assigned.

In an example where acquired imaging data are series of B-mode images, using voxelized reconstructed volumes may not always result in sufficient quality, for example when performing 3D segmentation tasks, when extracting slices from the reconstructed volume for visualization, or segmentation tasks. Thus, it may be advantageous to index a set of acquired 2D images with their positions, for example, 1D positions along a vein. In the example where the outcome metric is the shrinkage of a vein (e.g. the difference between the diameter of a treated vein before and after treatment, divided by the diameter before treatment) computed at different positions along the vein, an advantageous process may be to segment each or a subset of acquired images, to select a position on the image (preferably related to the vein, e.g. the center of the vein), to assign coordinates to the images (preferably, a 1D coordinate along an axis substantially corresponding to the main longitudinal axis of the vein, or a curvilinear abscissa along the vein) to each image, to preferably select a subset of images (to avoid having several images for the same coordinates), and to consider the subset of images and the associated coordinates. Then, for each position along the vein at which the outcome metric is to be computed, the local diameter is estimated based on the vein segmentation on the image. It may also be interpolated based on the vein segmentation in several images close to each position, especially if the displacement speed of the probe divided by the acquisition fram-erate is low compared to the desired spatial step for the outcome metric. The outcome metric may then be computed in each position along the vein, for example as the difference between the diameter before and after treatment, divided by the diameter before treatment.

Additionally or alternatively, it is possible to compute an outcome metric, for example the shrinkage of a vein, based on a 3D label map. In a preferred embodiment, the vein is segmented in each or a subset of the imaging data comprised in the acquired data. As known by the person skilled in the art, the image quality can be better in the acquired frames compared to images taken from a voxelized volume reconstructed based on the acquired frames. Then, a 2D label map is generated for each segmented frame. A reconstruction algorithm is then used to populate a 3D voxelized label map using techniques known by the man of the art (such as averaging for dealing with redundant data and interpolation to deal with missing data). Labels are preferably not simple integers to account for the complexity of the data (e.g., if a voxel is labeled as belonging to the vein according to one image and as not belonging to the vein according to another image, the label value can be averaged). Then, the diameter of the vein is extracted along the vein based on the label map, preferably in parallel planes, preferably equally-spaced along the vein.

If acquired data comprises ultrasound imaging data, they are preferably acquired with a hand-probe, which may offer better image quality than a probe embedded in a treatment head as, in the latter case, the probe may lack direct contact with a pa-tient's skin. In particular, nerves as well as a position of a vein are easier to image using a hand-probe.

Where an injection is performed (for example, a local or tumescent anesthesia) which induces a deformation or a collapse of the structure of interest, the acquired data before treatment is preferably captured before the injection, and the acquired data after treatment is preferably captured after the effect of the injection on the outcome metric has disappeared to avoid measuring effects of the injection itself. For example, in the context of the therapy of a vein, the post-treatment acquired data is preferably captured at least one hour after the injection, most preferably at least the day after.

Preferably, the outcome metric is computed for a collection of segments covering substantially all or at least a portion of the anatomical structure impacted by the treatment or a subsequent therapy.

For example, in the context of vein treatments, a treatment success may be evaluated based on the shrinkage of the vein, preferably defined as the difference between the diameter of the vein before and after treatment, divided by the diameter of the vein before treatment. A vein may also be occluded if the therapy damages the intima of the vein, which can result in an occlusive thrombus. In this case, the diameter is preferably the internal diameter of the vein (diameter of the lumen) or the external diameter of the vein (diameter of the zone comprising the vein lumen and the vein wall). It is particularly advantageous when the outcome metric is used to learn the best treatment parameters since denaturation of the collagen is usually an effect which is harder to achieve than simply damaging the intima, and is thought to lead to more durable occlusions. Alternatively, the diameter can be defined as the diameter of the section in which the blood can flow.

Acquired data are preferably captured with the patient substantially in the same position. Vein diameters are preferably measured in standing position.

Alternatively or additionally, an outcome metric may be computed based on shrinkage and/or incompressibility and/or absence of reflux and/or absence of flow. Additional effects may also be considered (for example, treatment duration, side effects, etc.). The variables reflecting the effects of the treatment can be combined into an outcome metric using methods known by the man of the art (weighted sums of the variables, nonlinear combinations...).

Computing an outcome metric may also include or consist in assigning a class to segments. For example, treatment slices can be attributed to a class representing local treatment success if the reflux is abolished in at least a portion of the vein and the shrinkage at the location of these treatment slices is greater than a certain threshold, for example 20 % or 50 %. Treatment slices can be attributed to a class representing local treatment failure if the shrinkage is smaller than a certain threshold, for example 20 % or 50 % and preferably if the vein is still open after the .treatment. Treatment slices not included in any of the two classes may for example be discarded from the subsequent analysis or attributed to one or several intermediate classes.

First and second imaging data can each be based on images acquired at several points in time. For example, the different variables used to compute an outcome metric may result from acquisitions performed at different timepoints. For example, the shrinkage may be evaluated in acute but the occlusion of the vein may be evaluated after a longer follow-up period (e.g. 1 month, several months, one year or several years). In particular, second imaging data may not be collected exactly at the same timepoint after treatment for all patients, but for example in the same range (e.g. 3 to 6 months after treatment, or more than 3 months after treatment).

Additional information not originating from medical imaging may also be considered for the outcome metric computation, such as patient satisfaction or an evaluation of symptoms severity (e.g. ulcer surface, occurrence of night cramps, and others).

Preferably, the treatment is divided in a series of treatment slices (positions or cross-sections along the vein, preferably equally-spaced, preferably substantially parallel with respect to each other, preferably substantially orthogonal to the main axis of the target, preferably characterized by an abscissa along an axis (e.g. representing the main longitudinal axis of a vessel) or a curvilinear abscissa and preferably a coordinate along another axis (e.g. representing a distance to the skin)). Preferably, the treatment slices are spaced by less than 5 cm, most preferably less than 1 cm. Preferably, 0 to 10 pulses are delivered in each treatment slice.

Preferably, the outcome metric is computed with a granularity which is compatible with the granularity of the therapy. The person skilled in the art will understand that granularity refers to the spatial scale or the level of details. For example, if a HITU treatment consists of a series of pulses delivered in consecutive transverse slices of the vein spaced by N mm, the outcome metric is preferably computed every N mm at locations corresponding substantially to the transverse slices used for treatment (treatment slices). This is advantageous because it enables to directly relate the outcome metric to the local treatment parameters. Additionally or alternatively, the outcome metric may be computed at a bigger scale, preferably for vein segments measuring a multiple of N mm in length. Preferably, N is comprised between 1 and 10. Preferably, the segments are centered on the locations of the treatment slices comprised in the segment. Additionally or alternatively, the outcome metric can be computed at a finer scale.

Additionally or alternatively, other outcome metrics can be derived at the scale of the whole structure of interest (e.g. absence of reflux in a vein, occlusion, volume of a tumor) and preferably combined with local outcome metrics.

In the context of the therapy of varicose veins with HITU, the treatment subject to the computation of the outcome metric may also be patient positioning. Indeed, machine learning analysis have shown that the depth under skin of the vein in a treatment position is a predictor of shrinkage. The smaller the depth of the target under the skin, the less power is allowed by the treatment device to avoid skin burns. However, the depth under skin of the vein in treatment position, when the patient is in a substantially lying position and the HITU treatment head presses on the leg, can be difficult to estimate based on the depth under skin of the vein during a sonographic examination with the patient in standing position. Thus, it is advantageous to compute an outcome metric as the difference of the depth under skin in treatment configuration and in standing position. In this case, the treatment preferably consists in placing the patient substantially in treatment position (which may include specific positioning such as Trendelenburg positioning) and/or applying the HITU treatment head onto the patient and/or applying pressure onto the vein as in treatment.

In the context of the therapy of at least a tissue or an organ (preferably the therapy of at least a varicose vein with HIFU), the treatment subject to the computation of the outcome metric may also be patient positioning and the outcome metric may be difference in the position of the vein in an image.

Typically, first imaging data acquisition may consist in acquiring a series of medical images (preferably B-mode images) from a vein on which a therapy (preferably a HITU therapy) is to be delivered and their associated locations and/or orientations. Locations and/or orientations may be acquired by methods known by the person of the art, including optical tracking, electromagnetic tracking, sensors of a robot, in particular a robotic arm holding the imaging probe serving to acquire the medical images.

In a first preferred embodiment, the medical images of the first imaging data are captured with an ultrasound probe, e.g. a hand probe, and the position of the probe is known based on optical tracking or robotic sensors. The probe may he held by a robot.

- The probe may be in substantially direct contact with the skin (e.g. there may be acoustic gel to improve acoustic coupling, but the distance between the probe and the skin is small, e.g. less than one millimeter).

In a second preferred embodiment, the medical images of the first imaging data are captured with an ultrasound probe embedded in the treatment head which will serve to deliver a therapy in the vein. In this case, the ultrasound probe may not be in direct contact with the skin (e.g. a membrane filled with liquid may be mounted on the treatment head, in which case, the ultrasound probe may be separated from the skin by several millimeters of liquid).

Preferably, at least part of the first imaging data is acquired with a low pressure exerted onto the vein (i.e. a pressure which enables to see the vein on the image, preferably a pressure which does not completely stop blood flow into the vein for example). The pressure exerted onto the skin may be below 10 mbar).

Preferably, the patient is in position for the therapy (e.g. in substantially laying position, such as in Trendelenburg position). Preferably, if an injection (e.g. a local anesthesia) is to be performed for the therapy, at least part of the first imaging data is captured after the injection.

Then, second imaging data acquisition may consist in acquiring a series of medical images (preferably B-mode images) from the same tissue or organ (e.g. a vein) in the same region.

Preferably, at least part of the second imaging data is captured with a treatment head (preferably the HIFU treatment head used to deliver the therapy). Preferably, at least part of the second imaging data is captured before the therapy is delivered, but after the treatment head is positioned for delivering the therapy, and preferably with an applied pressure similar to the pressure which will be applied during the therapy itself. Preferably, the position of the treated tissue or organ (e.g. a vein) and/or other structures just before or when the therapy is delivered is the outcome metric, and the treatment consists in positioning the treatment head with the appropriate pressure.

In particular, part of the second imaging data may be captured after part of the first imaging data. In a preferred embodiment, in an example where the therapy is a HIFU treatment of a vein and the HIFU device comprises a robot with a limited robotic range which does not enable to treat the whole vein, a first step may consist in positioning the HIFU treatment head so that the vein is within the B-mode image of the treatment head. Preferably, the vein is substantially centered in the image, and the images are substantially orthogonal to the main axis of the vein. The first imaging data may be a series of B-mode images captured along a first vein section with the treatment head and preferably a low pressure setting. Preferably, all images captured are captured with position data at least enabling to locate the images along the vein (e.g. known from a robot holding the treatment head). Then, the treatment head may be placed in a first treatment slice, and the pressure is preferably increased to collapse the vein. At least an image is captured, preferably before the first pulse is delivered, and added to the second imaging data. When the treatment head moves to a second treatment slice, at least another image is captured, preferably before the first pulse is delivered, and added to the second imaging data. Once the first section is treated by the HIFU device, the treatment head is repositioned on a second vein section. A series of B-mode images are captured along a first vein section with the treatment head and preferably a low pressure setting, and added to the first imaging data. Thus, in this example, some images from the first imaging data are more recent than some images from the second imaging data. Then, the treatment head is placed in a treatment slice, and the pressure is preferably increased to collapse the vein. At least an image is captured, preferably before the first pulse is delivered on this treatment slice, and added to the second imaging data.

The process may be repeated, preferably until completion of the HITU treatment. If an action occurs (e.g. local anesthesia due to pain), , the first imaging data may be considered not suitable for an outcome metric computation, e.g. if the objective of the outcome metric is not to quantify the effect of this action,. Thus, new images are preferably captured with a low pressure, and added to the first imaging data. It shall be understood that, in this example, the increase in pressure also substantially modifies the aspect of the vein, but it is part of the treatment whose outcome is quantified by the outcome metric.

The outcome metric is then computed as the difference in the position of the vein or other structures visible in the images (including deformation) for at least two pairs of images comprising one image from the first imaging data and one image from the second imaging data captured at substantially the same position along the'vein.

In a preferred embodiment, further imaging data may comprising images corresponding to an intermediate state between first and second imaging data (e.g. medical images captured at a pressure which is lower than the pressure applied in treatment, but substantially collapses the vein) may be considered. Having images corresponding to such intermediate state is particularly advantageous when training a machine learning model to predict the outcome metric based on the first imaging data.

In another preferred embodiment, the first imaging data com-prises two images in substantially the same state (e.g. uncompressed vein), but captured with different probes or image modalities (e.g. a hand-probe and a treatment head). This is particularly advantageous when training a machine learning model to predict the outcome metric based on the first imaging data.

The outcome metric may also be computed based on several acquisitions at different timepoints before and/or after treatment, aggregated by a function.

It is also conceivable to compute several outcome metrics representing different time points (e.g. from acquired data before treatment combined with acquired data at different time points to track the development of an outcome metric over time). Additionally or alternatively, acquired data from more than two timepoints may be combined to calculate an outcome metric (e.g. acquired data before treatment and a data set of averaged acquired data from several time points). For example, an outcome metric could be computed as the shrinkage of a vein one year after the treatment with addition of a percentage (e.g. 10 %) of the shrinkage of the vein one week after treatment. If the outcome metric is to be correlated with treatment characteristics in order to select the most efficient treatment parameters, this may for example enable to favor the.treatment parameters having resulted in a long-term success but also in an acute shrinkage.

Preferably, the algorithm is trained to predict an outcome metric (for example, the vein shrinkage) at a local scale (preferably at the scale of the treatment slice). The algorithm may further consider fixed features, which are invariable for a particular patient (e.g. sex and/or age on the day of treatment) and/or variable features which can be adapted by a user (e.g. HITU power, number of pulses, etc.), and/or monitoring features which occur as a result of a treatment (e.g. hyperechoic marks, temperature field, etc.).

The outcome metric may be computed based on data collected at different timepoints, the time interval between timepoints being adapted to treatments and body tissues which are evaluated. For example, in the context of the thermal occlusion of veins, long-term studies are conducted with a follow-up of several years (for example, 2 years, or 5 years). Data can also be collected after shorter follow-up durations (for example, 3 months or even a few days) to train the algorithms. Then, the algorithms are preferably retrained oncelong-term data is collected. Vein shrinkage data can even be collected immediately post-treatment if no injection was performed in the surroundings. Tumor shrinkage (for example in breast fibroadenoma or thyroid nodules) is preferably observed after several months (preferably after 1 months, most preferably after 3 months).

The outcome metric is preferably the target of the machine learning algorithms. Several success or outcome metrics may be combined or used sequentially for different machine learning problems in a single software.

In particular for the treatment of veins, fixed features may include sex, age, height, weight, BMI, profession requiring standing position, comorbidities, current treatments (e.g. anticoagulants), number of pregnancies, reflux type (systolic or dias-tolic), pre-treatment vein diameter in standing position, pre-treatment volume of a tumor, history of other treatments performed on the target, number of tributaries of a vein, and/or location of fibrotic tissue in the vicinity of the target (resulting from a previous treatment for example).

Variable features may include pulse date and time, pulse types, pulse power, pulse frequency, pulse duration, treatment head position, and/or pressure exerted onto the tissue.

Further variable features may be considered, for example the occurrence of an adjunctive therapy (e.g. sclerotherapy in a vein), the occurrence and/or volume of infiltration (e.g. local anesthesia or injection of another fluid) and/or a length or a percentage of the vein length which was treated. This is particularly advantageous where the goal of the treatment is an occlusion of the vein, and/or where input data is a sequence of parameters describing the treatment performed in treatment slices, in particular a sequence containing the treatment parameters used in all treatment slices for each vein. In some cases, some treatment slices may not have any delivered pulses.

Monitoring features may include history of previous pulses parameters, number of pulses delivered on the current treatment slice, total energy delivered on the vein since the beginning of the treatment, pre-pulse and/or post-pulse images of previous pulses, and/or extracted information (e.g. HEM area and/or HEM location with respect to the vein), and/or live B-mode image.

Note that the classification of the features into the different types of features is only given as an example. For example, the occurrence of local anesthesia may be assigned as a fixed feature if the doctor decides to avoid it (e.g. to be fully non-invasive) or, on the contrary, wants to do it (e.g. to improve patient comfort). Alternatively, it may be considered as a variable feature (e.g. if the doctor considers it as an option to increase the distance between the skin and the target, or in case patient feels pain).

"Treatment parameters" will refer to features which can be at least partially controlled, i.e. variable or monitoring features. "Treatment characteristics" will refer to all types of features which are considered by the system or the computer program or the user, thus also including fixed features. For clarity, the distinction between treatment characteristic and treatment parameter is made herein. However, it will be understood by the person skilled in the art that any treatment parameter may be a treatment characteristic. To the extent treatment characteristic are at least partially controllable, in particular when referring to variable and monitoring features, the terms treatment characteristic and treatment parameter may be used interchangeably. For example, a pulse power-may be treatment characteristic and also a treatment parameter. By contrast, a weight or sex of a patient may be a treatment characteristic considered but would not typically be a treatment parameter.

It is possible, in some embodiments, that a first machine learning algorithms shows that one feature (e.g. sum of the HEM areas resulting from all pulses performed in a given segment) is a good predictor of treatment success. Thus, a second machine learning algorithm may be used to evaluate a correlation of the feature on a segment level (e.g. HEM area and treatment outcome on a segmental level).

Where the training of a machine learning algorithm is performed at a given spatial scale, e.g. the scale of the treatment slice, spatially invariable features such as the age of the patient may be duplicated for each slice, e.g. assumed to be constant.

Features varying at a bigger scale (e.g. the occurrence of injection, which is likely to impact several treatment slices) may be duplicated for each slice, e.g. assumed to be constant.

By contrast features which may vary at least at a smaller scale, for example (e.g. power delivered during HITU pulses), may require further processing. Preferably, such parameters are aggregated. For example, a power can be aggregated by taking the average power over multiple or all treatment pulses delivered in a given segment. Additionally or alternatively, further information may be acquired or extracted. For example, heat maps formed by 2D pictures containing the power delivered in each point of the treatment slice may be used. Additionally or alternatively, information may be stored in lists with one entry for each pulse.

Preferably, the imaging data is obtained by an acquisition of at least an anatomical structure of interest. Imaging modalities are known by the skilled person and include ultrasound imaging modalities such as B-mode, duplex, elastography, and other imaging modalities.

The position and orientation of the acquisition system, for example an ultrasound hand probe, can be determined based on a tracking system, for example and electromagnetic tracking system, or an optical tracking system, or an inertial tracking system. Additionally or alternatively, the acquisition system may be fixed on a robot and the position and orientation of the acquisition system may be known by the sensors of the robot.

The anatomical structure of interest preferably comprises an incompetent (i.e. refluxing) vein, or a tumor (for example, a thy-raid nodule or a breast fibroadenoma, or a malignant tumor). The acquisition is preferably performed with the patient in a position which is suitable for clinical evaluation of the pathology. For example, in the context of chronic venous insufficiency, the patient is preferably in standing position. Other considerations such as patient comfort can also be taken into consideration, since it may reduce the occurrence of movements. For example, the acquisition for a thyroid nodule is preferably performed with the patient in a lying position. The acquisition can be performed in a position suitable for a therapy, or in a different position. Acquisition may result in at least an acquired data. Preferably, all acquisitions serving to compute an outcome metric are performed with substantially the same patient position.

Alternatively, if the change of position of the patient is part of the treatment itself, the acquisitions serving to compute the outcome metric are performed with the patient in different positions. For example, the treatment may consist in positioning the treatment from standing to substantially lying position and to position a treatment head, e.g. a HITU treatment head.

Slices may be extracted from the reconstructed volume and the structure of interest can be manually or automatically segmented. An advantage of performing the segmentation on extracted slices is that their position in space can be chosen to facilitate a 3D model construction (e.g. a 3D mesh). Preferably, the extracted slices are parallel to each other, to facilitate 3D model construction.

Preferably, the extracted slices are orthogonal to an axis of the longest dimension of the structure of interest. Preferably, if an ultrasound therapy is to be or was delivered on the structure of interest, the extracted slices are substantially parallel to the treatment slices.

Additionally or alternatively, segmentation may be performed on acquired images. A reconstructed model may be constructed based on the segmented acquired images. For example, for a vein, the segmented contours can be resampled to comprise the same number of points, and a simple quad mesh can be generated, with each quad comprising two points from a contour and the two corresponding points of a neighboring contour.

An advantage of performing the segmentation on the acquired images is that the image quality is generally better than reconstructed images.

The system and methods according to the invention, in particular when registration is performed, are particularly useful for patient follow-up at different points in time. For example, where a physician wants to follow the evolution of a pathology (e.g. chronic venous insufficiency) or the outcome of a therapy (e.g. the volume reduction of a tumor or the shrinkage or occlusion of a vein).

For chronic venous insufficiency, at least part of, preferably all, the acquisitions generating medical images serving to compute the outcome metric are preferably performed with a hand-probe and with the patient in a standing position. The user preferably scans a portion of the patient's leg comprising at least a portion of a vein. B-mode/MR imaging enables an assessment of a diameter of the vein along its course. Scanning may be repeated after a therapy, thus enabling to see if the vein has shrunken due to the therapy (e.g. after a thermal ablation) or if it is occluded. Doppler mode may be used to assess whether an occlusion has occurred. Compressibility may also be assessed, in B-mode for example, by applying pressure onto the vein, with hand-probe during the acquisition for example.

Preferably, the memory is a non-volatile memory such as an SSD, a magnetic hard drive, a compact disc, or a USB flash drive. In some embodiments, the memory is a volatile memory, wherein the data can be transmitted via an interface to a non-volatile memory.

Preferably the method acquiring the position and/or orientation data serving for the registration of the first and second imaging data is also adapted to capture positionand/or orientation data serving to spatially register at least one local treatment characteristic with the outcome metric or local treatment outcome.

For example, a position and orientation of images may be captured with an optical tracking system. Optical markers (for example passive markers or active markers) may be placed at a preferably fixed or reproducible position with respect to patient anatomy. For example, in the context of HITU treatments, preferably HITU treatments of varicose veins in the leg, optical markers are preferably placed:

- on a hand-probe which serves to acquire acquired data before and after treatment, and/or
- on a kneepad, preferably comprising a hole for the patella for a more reproducible repositioning during follow-up visits, and/or
- one or several other wearables which are substantially fixed with respect to the anatomical structure of interest, where the wearables can preferably be removed and reliably placed at substantially the same position (e.g. with holes or marks serving to control the position and orientation), and/or
- on a stage serving for the acquisitions, and/or
- placed directly on the patient anatomy (e.g. using one or several disposable electro cardiogram patches or other skin-compatible adhesives)
- on the treatment head which serves to apply the HITU energy.

Such marker placement is advantageous because it can allow the position of the images to be known with respect to the knee (or another anatomical location), thus allowing direction registration (or registration initialization) of the first and second imaging data. Moreover, the recording of the position and/or orientation of the HITU treatment head with respect to the same anatomical location enables to relate at least one local treatment characteristics to the local treatment outcome. The vein may be, for example, a great saphenous vein or an anterior accessory saphenous vein or a small saphenous vein or another truncal vein or a perforator.

Additionally or alternatively, the registration of the first and second imaging data and/or the registration of one or both imaging data with at least one treatment characteristic is performed or initialized based on anatomical landmarks.

For example, landmarks may be anatomical structures visible in the imaging data, and preferably during treatment (for example, via an ultrasound probe embedded in a treatment head) or just before and/or after treatment during a dedicated acquisition sequence (for example performed with an ultrasound probe embedded in a treatment head or a hand-probe) performed with the patient in substantially the same position as in treatment.

In the context of the Great Saphenous Vein (GSV) or Anterior Accessory Saphenous Vein (AASV), the end of the vein, i.e. the sapheno-femoral junction (SFJ) may not always be a sufficiently precise landmark since it is not always easily accessible, especially on patients with high body-mass indices. However, in some embodiments, the sapheno-femoral junction may be used as landmark.

Preferably, junctions of the target vein with other veins (epigastric, posterior saphenous vein (PSV), AASV or GSV, tributaries, and/or perforators) serve as landmarks.

Global landmarks (i.e. landmarks which run along at least a significant portion of the vein, e.g. the femoral vein or fascia, in particular over at least 20%, preferably 50% of a vein) can also provide some information.

For example, a distance can be computed between the GSV and the skin, or between the GSV and the femoral vein. Registering these curves can help registering two acquired datasets.

Tributaries (veins branching from the target vein) for example can be considered either global landmarks if considered along at least a portion of their course, or as local landmarks if limited information is considered (junctions, escape points...). The 3D conformation of some structures (e.g. veins) can also serve for registration.

Local landmarks are often more reliable than global landmarks. Local landmarks can either be identified manually by the user or automatically detected by an algorithm. For example, for a GSV, lymph nodes or junctions with tributaries, epigastric, AASV, PSV) can be used as landmarks.

In particular, the junctions with the veins are by definition bound to the vein, thus, their localization is usually robust to the exerted pressure, although applying pressure may make them invisible.

Additionally or alternatively, the registration of the first and second imaging data and/or the registration of one or both imaging data with at least one treatment characteristic is performed or initialized based on external fiducials or a combination of anatomical and external fiducials.

External fiducial(s) may be added by placing at least one sensor and/or marker on the patient's anatomy.

A sensor may refer to an active device. A marker may be one or a set of visually perceptible landmarks (such as balls used for motion capture, or a QR code), or an electromagnetic sensor or any other passive device which location can be stored in a computer to serve as reference. Preferably, the external fiducial is removable but it can be reliably placed at approximately the same position using to a prominent structure (such as the knee or the ankle), which preferably does not substantially move with respect to the vein.

It will be understood that markers and sensors may be used in combination or individually. For example, a motion sensor may be used in combination with a visual marker. While the latter may be tracked by camera, the former assists in detecting when (and in which direction) a movement happens. Alternatively, only markers may be used, or only sensors.

Typical markers are known in the art and in particular include balls used for motion capture and QR codes.

Typical sensors are known in the art and in particular include optical tracking cameras and electromagnetic tracking devices. Typically, for the GSV in its proximal half (or other targets in the thigh), it is beneficial to place a sensor and/or marker on the knee using a kneepad, the sensor.and/or marker being preferably on the upper (proximal) part of the knee, or slightly more proximal.

Similarly, for targets located in the calf, a sensor and/or marker can be added on the knee (preferably on le lower part of the knee or below), or on the ankle.

It will be understood that landmarks as described above in the context of veins, may also be used for any other treatment or in combination with any system according to the invention, in particular with other structures of interests. For example, in the context of thyroid nodules, the carotid artery can serve as global landmark and the thyroid isthmus can serve as local landmark.

The registration process is preferably done based on the available landmarks as this may provide a more robust registration because of their invariability after a therapy or physiological changes, especially over long periods of time.

Preferably, the registration is rigid, but non-rigid registra-tion can alternatively be performed.

When imaging data of a structure of interest and at least a landmark is provided, it is generally possible to register two acquisitions of a tubular structure (such as a vein). Preferably, the landmark is located at one end or after the end of the structure of interest, which advantageously provides unambiguous information about the location of the current plane.

The system may further comprise at least one control unit for controlling an imaging unit and/or a treatment device.

Preferably, the computer device is further adapted to receive and save treatment characteristics linked to the outcome metrics in the memory. "Linked" may be understood, for example, where the outcome metric is the shrinkage of a treated vein treated by HITU, characteristics of the vein (e.g. diameter and/or depth under skin) may be "linked" to the outcome of the treatment, as well as technical treatment characteristics (e.g. power, duration, frequency of the HITU pulses, number of pulses delivered, location of the HITU pulses, and/or images captured) and treatment characteristics related to a clinical practice (e.g. occurrence of injection, volume of injected fluid, Trendelenburg positioning of the patient, and/or adjunctive- therapy). Moreover, characteristics of the patient such as patient's age, sex, comorbidities, medical history may also be "linked" to the outcome metrics. Other characteristics, such as the laterality of the vein (which leg the vein belongs to, i.e. left or right, which may influence the positioning of the patient with respect to the device), the experience of the practitioner (expressed in number of procedures performed, for example), the gain used in ' B-mode images during treatment, the room temperature (since abnormal temperatures may affect the proper functioning of hardware components) may also be considered as "linked" to the outcome metric.

In particular, the computer device may record treatment characteristics such as power, number of pulses, duration of pulses, frequency, and/or pressure exerted on the tissue which were used, while or before the second imaging data was obtained. Preferably, the treatment characteristics are assigned to spatial locations (preferably coordinates, or treatment slices, or portions of the target where an outcome metric is to be computed). This enables to relate the global and/or local treatment characteristics to the outcome metric. Treatment characteristics may be automatically recorded by the system using sensors (e.g. delivered acoustic power during a HITU treatment), but they may also be manually input, e.g. by the user (e.g. sex of the patient).

For example, if the outcome metric is the shrinkage of a vein, the treatment is a HITU treatment, one of the treatment characteristics may be the power of the HITU pulses. For example, the power of the HITU pulses may be aggregated at the scale of the treatment slice, for example by taking the average power of all pulses delivered in each treatment slice. It will be understood that the treatment characteristics can be aggregated at another scale, and even that different treatment characteristics can be aggregated, computed or recorded at different scales. If the position and/or orientation of the treatment head with respect to the vein is known, this enables to relate treatment characteristics with values of the outcome metric. In a simple example, this enables to relate the local shrinkage in each treatment slice with the average power of the HITU pulses delivered in each treatment slice.

It is especially advantageous to relate treatment characteristics comprising at least one local treatment characteristic to an outcome metric which describes the local outcome of a treatment since, for some indications (e.g. the coagulation of veins), and for some treatment modalities (e.g. HITU treatments), the local outcome of the treatment may vary significantly within the same target. For example, the depth under skin of a Great Saphenous Vein may vary from typically 25 mm to 10 mm along its courses, which may change the conditions of propagation of a HITU beam thus, for example, the optimal frequency or the maximum acoustic power which can be tolerated by a patient's skin. This can result in very different local treatment outcome along the vein, with some portions being completely coagulated and some portions being not significantly treated. Thus, relating treatment characteristics including at least one local treatment characteristics and the local outcome is advantageous to optimize the outcome of the treatment.

Treatment characteristics refers to physical or technical treatment characteristics (such as power, number of HITU pulses, occurrence of injection, concentration of a sclerosant) and/or other data, preferably including data related to the characteristics of the target (e.g. type of vein, local depth under skin of the target), the characteristics of the patient (sex, age, weight, height...) and/or its medical history (e.g. previous treatments, anticoagulants, comorbidities). Any of these data may be stored in a non-volatile memory which is part of the system.

Preferably the segments in which the outcome metric is computed are defined after the treatment has been delivered, preferably based on the spatial repartition of the treatment. For example, the segments may correspond to the treatment slices of a HITU treatment. This is advantageous because it is usually easy and relevant to interpolate acquired data or intermediate data while it does not necessarily make sense to interpolate treatment characteristics. For example, if the outcome metric is the shrinkage of a vein and the treatment is a HITU treatment, the diameter of the vein can be easily interpolated to the positions of the treatment slices. For example, it is often reasonable to consider that, if the diameter of a GSV is 8 mm on an image taken 15 cm away from the sapheno-femoral junction, and 9 mm on an image taken 15.4 cm away from the sapheno-femoral junction, the diameter of the vein is 8.5 mm at a position located 15.2 cm away from the sapheno-femoral junction. Contrarily, interpolating treatment characteristics such as the mean area of the hyperechoic marks observed after HITU pulses delivered in a treatment slice, or the number of pulses delivered in a slice may result in data which are more difficult to interpret or less relevant, for example because of threshold effect.

Preferably, the system comprises a treatment device. The treatment device may comprise or consist of a HITU transducer.

Preferably, the control unit may be adapted to record the treatment characteristics and transmit said treatment characteristics to the computer via the interface, preferably automatically.

Preferably, the treatment characteristics are recorded with respect to position and/or orientation data enabling to spatially relate the treatment characteristics to local treatment outcome.

The control unit may be adapted to control the treatment device as well. Particularly preferably, the control unit is adapted to perform the control of the treatment device and/or transmission of the treatment characteristics automatically.

As described above, the position of a treatment head may be a variable feature used in the methods and systems according to the invention. It may therefore be advantageous, in certain embodiments, to determine the position of the treatment head with respect,to a treatment location, a region of the body to be treated, and/or a treatment slice. Typically, a software is used which tracks a position of the treatment head and registers a coordinate system used for the treatment head position with the pre-treatment imaging data. To register the pre-treatment imaging data and the coordination system of the treatment head, a registration acquisition is preferably done once-the patient is in treatment position and landmarks serve for the registration.

Skin marking may be used as landmarks, in which case a camera can be used as a sensor to relate the position of a treatment device and/or of an image acquisition device to the mark on the skin.

Determination of the position of the treatment head may be achieved by mounting the treatment head on a robot which automatically tracks the position. Additionally or alternatively, an electromagnetic marker and/or an optical marker may be used and tracked with a separate tracking device.

In some embodiments, two or more ways of tracking the position of the treatment head may be combined, for example in order reduce the noise during acquisition by combining the information (e.g. by averaging) the information coming from the two tracking means.

In particular, a treatment head which is mounted on a robot may be combined with an optical or electromagnetic tracking system in order to track the position of the treatment head accurately even in cases where the entire robot including the treatment has to be moved. This may be necessary in cases where the robot is unable to reach certain positions necessary for treatment. In such cases, a user may move the robot itself to reach such positions. However, the recorded position by the robot may have to be corrected in such cases, for which other systems may be used.

Preferably, the system further comprises an imaging device. The imaging device is controllable by a control unit. The control unit for controlling the imaging device may also be adapted to control the treatment device, i.e. the system may comprise one control unit for both the imaging and the treatment device. Alternatively, the system may also comprise a first and a second control unit, wherein the first control unit is adapted to control the imaging device and the second control unit is adapted to control the treatment device. The imaging device is prefera-bly a B-mode imaging device. The computer may be adapted to receive the first and second imaging data from the imaging device via the interface.

Preferably, the imaging device comprises at least a first and a second imaging probe. The first imaging probe may be a hand probe.

Preferably, the second imaging device forms a part of the treatment device.

Particularly preferably, the first imaging probe is adapted to gather the first imaging data and/or the second imaging probe is adapted to gather the second imaging data.

Preferably, the computer device, in particular one of the first and second computer device, is adapted to register the first and second imaging data.

Preferably, the system further comprises means for estimating an applied pressure on the body region and/or patient's tissue. The means for estimating an applied pressure may comprise any one of a force sensor and a contact sensor. A contact sensor may measure the contact area between, for example, the treatment device and/or imaging device and the patient's skin. The contact area typically increases with increases pressure, thus allowing for estimation of a contact force. It will be understood that the force applied to the body region may be measured as an absolute number or qualitatively. For certain applications, it is sufficient to determine a qualitative pressure level to allow for comparisons between different treatment or imaging steps. In some embodiments, the means for estimating an applied pressure may be formed by the imaging means or a second imaging device. The applied pressure is determined by analysis of imaging data, for example by determining when a vein collapses or the thickness/compression of other tissue types. Particularly preferably, the system also comprises means for applying a pressure on a body region while imaging. Where means for applying a pressure are present, the system may optionally be adapted to record the applied pressure and save it as a treatment characteristic.

The invention is further directed to a method of gathering treatment data and/or evaluation treatment data. Preferably, the method is performed using a system as described above. The method comprises obtaining first imaging data of a body -region representing a state before or without a treatment. Furthermore, second imaging data of the body region is obtained, wherein the second imaging data represents a state after or during a treatment. The used treatment characteristics are also obtained and may comprise any of power, number of pulses, duration of pulses frequency, and/or pressure exerted on the tissue.

The data may optionally be saved on a memory device.

A first and second prepared models are created, wherein.the first model preferably represents the body region before treatment and the second model represents the body region during or after a treatment has been conducted. Particularly preferably, the first and second prepared models are created by a computer associated with a treatment and/or imaging device and/or the system as described above.

Additionally or alternatively, it is also conceivable to collect imaging data comprising image positions captured by 3D trackers to a server, where the first and second prepared models are created.

Furthermore, the method optionally comprises registering the first and second imaging data and/or the first and second models.

Further optionally, the at least one outcome metric which represents a change between the first and second prepared models is determined. In particular, the outcome metric may represent a change in a diameter of a vessel, preferably an inner or an outer diameter of a vessel.

The outcome metric is determined for at least two segments of the body region.

Additionally, the method may comprise a step of collecting at least one treatment characteristic and corresponding position and/or orientation data. This may enable to spatially relate the treatment characteristics to local treatment outcome. For example, a pulse duration and/or a number of pulses may be saved with respect to a certain treatment location. The location data is adapted to be assigned to a treatment segment and/or an outcome metric.

Preferably, the outcome metric represents a change in a tissue characteristic before and after, i.e. due to, the treatment. For example, the success metric may be a change between an internal (or external) diameter of a vessel before and after a treatment has occurred.

Preferably, the second imaging data represents a state where a patient is in position for a therapy (e.g. in substantially lying position, such as in Trendelenburg position) and/or a treatment head is applied to a patient and/or pressure is exerted on the tissue. The outcome metric may then represent a change in distance between a patient's skin and a tissue to be treated compared to when the patient was in the position which the first imaging data represents (e.g. in substantially standing position).

As a result, it can be possible to train a model to predict the depth under skin of a tissue when a patient is positioned for a therapy based on the depth under skin of the tissue when the patient is in another position which is commonly used for examinations (e.g. standing position) and preferably other features (such as patient's height, weight, age). This is advantageous for example for patient selection for HITU therapy of veins. Indeed, depth under skin may influence HITU therapies since the power which can be delivered without damaging the skin for a given frequency and pulse duration may depend on the depth under skin. Thus, the model may help to determine if a certain patient may be treatable based on a simple examination performed in a usual setting (e.g. with a hand-probe and the patient in standing position).

In general, the depth under skin (either captured in standing position, or in treatment position, or the depth under skin in treatment position estimated based on the depth under skin captured in a usual examination setting) may be considered when training models to predict an outcome metric related to the effect of a HITU treatment.

Preferably, the method further comprises the step of determining a correlation between the outcome metric and at least one of the treatment characteristics, the at least one treatment characteristic preferably comprising a treatment parameter. Additionally or alternatively, a model is determined which allows to associate outcome metrics to at least one treatment characteristics. The model may for example be a mathematical and/or statistical model and/or an artificial intelligence model and/or the output of an inferential statistical analysis. The determined correlation may be part of the model.

The at least one treatment characteristic may be any parameter associated with a patient, in particular a body mass index (BMI), sex, and age of the patient. Additionally or alternatively, treatment characteristics may include a mean pre-treatment diameter of a vein or pre-treatment volume of a tumor, a number of pulses delivered, a mean power used over all pulses delivered, and/or a mean hyperechoic mark (HEM) area over all pulses delivered. It will be understood that any treatment characteristic disclosed herein may be used to determine a correlation.

The invention is further directed to a method of determining at least one treatment parameter for treating a patient. The method comprises obtaining imaging data of a body region to be treated. For example, a model may be obtained, in particular a reconstructed volume or a three-dimensional model. From the imaging data, at least two segments of the body region are preferably selected. Furthermore, at least one value of one treatment parameter is automatically selected. The selected value is suitable for achieving an optimized value of an objective function. Preferably, the optimization takes into account at least two segments, e.g. the two segments selected from the imaging data. It will be understood by the person skilled in the art that a "value" may for example refer to a single value, a set of values or one or several ranges of values.

Optimization in this context may in particular mean the minimization ore maximization of an objective function, i.e. the outcome metric is optimized such as to lead to a local or global optimum (minimum or maximum) of said objective function. The treatment characteristic may be automatically applied and/or proposed to the user if it is a treatment parameter.

Preferably, the objective function at least represents clinical success on the scale of the structure of interest. For example, computing the objective function based on the outcome metric may be performed based on a machine learning model trained to predict the clinical success (at the scale of the structure of interest) based on an outcome metric and/or treatment parameters.

Additionally or alternatively, the outcome metric to be achieved for at least two segments is defined as part of the method.

For example, an objective function may also relate to a treatment duration which is to be minimized. In some instances, the treatment characteristics may be selected to treat one segment with higher energy and/or a longer duration than another one, because this may minimize the total duration of the treatment while achieving the same treatment effect compared to treating both segments with the same parameters. To this effect, an outcome metric which is not minimized may be accepted if a sufficient minimization is reached. This may be the case where longer treatment durations continuously reduce the outcome metric, but the incremental therapeutic effect becomes secondary to the cost of the treatment duration (e.g. discomfort for patient and/or damage to tissue and/or medico-economic considerations).

It will be understood that the method according to the invention takes into account at least two segments which will, in practice, often lead two different treatment characteristics being selected for each segment. It is also possible, however, that in some instances the same treatment characteristics lead to an optimized objective function and can thus be selected.

Preferably, the at least two "segments" for which at least a treatment parameter is determined are of the same nature and size (e.g. a treatment slice) as the "segments" which were used for the outcome metric computation for which a correlation was determined. However, it may not be the case. For example, a correlation can be determined between the vein diameter and the number of HITU pulses and the vein shrinkage at the scale of the treatment slice. Then, a "segment" for which at least a treatment parameter is determined can encompass several treatment slices, i.e. several "segments" used for the outcome metric computation (e.g. the vein diameter may be averaged over a distance corresponding to several treatment slices).

The optimization of the objective function may be based on a mathematical model and/or an algorithm stored on a memory de-' vice.

For example, if the treatment is a HITU treatment of veins and the treatment parameter is the number of pulses per treatment slice (or the number of pulses inducing an HEM per treatment slice) and treatment characteristics captured before treatment include the local vein diameter, the algorithm used to select a value of the number of pulses to deliver per slice- may comprise dividing the local vein diameter by a suitable constant.

Additionally or alternatively, if the local vein depth under skin is captured before treatment, and treatment parameters include pulse type (characterized by frequency and/or power and/or duration of the pulse, for example), the pulse type may be chosen based on thresholds on the local depth under skin. If both a number of pulses per treatment slice and pulse types are defined, the constant value by which the local vein diameter is divided to compute the number of pulses per treatment slice preferably depends on the selected pulse type in the treatment slice. The rules defined hereinabove may or may not result from a machine learning analysis performed based on data according to the invention. It will be understood that rules and parameters determined may be adapted by a user . For example, the constant by which the diameter is divided to compute the recommended number of pulses or the recommended number of pulses achieving a certain effect, e.g. a HEM, or the recommended number of treatment sites in a treatment slice may be manually modified by the user or may be automatically modified if some treatment characteristics of treatment parameters (e.g. the pulse type) is changed.

The number of pulses or treatment sites (treatment locations at which one or several pulses are to be delivered) per slice may be displayed to the user in each treatment slice, or automatically delivered. It is particularly advantageous to combine with a feature enabling to deliver several pulses or to treat several treatment sites in a row in the same treatment slice, preferably with a fixed spacing. In a preferred embodiment, the optimal number of pulses or treatment sites in a treatment slice is proposed by the software, or is manually selected by the user.

In a further aspect of the invention, in the context of the treatment of one or a collection of vessels with HITU, a method may consist in:

- collecting medical images (preferably using at least B-mode images using an ultrasound hand-probe) of at least one of the vessels to be treated in a first position, preferably with the patient in a standing position. In a preferred embodiment, a tracking system (e.g. an optical tracking system) tracks the position of the images (e.g. by tracking the ultrasound hand-probe). Preferably, the position of the images is known with respect to the patient: e.g. in the context of optical tracking, markers can be placed directly on the patient anatomy (e.g. using one or several disposable electro cardiograms patches or other skin-compatible adhesives) or on wearables (e.g. a kneepad) and their position and orientation may also be captured by the tracking system. These markers are preferably substantially at the same position with respect to the target of the treatment during pre-treatment medical images collection and during the subsequent treatment. Preferably, this is achieved by placing the markers on one or several anatomical segments which do not move with respect to at least part of the target (e.g. on the thigh if the target is a vessel running in the thigh at least along a portion of its course) and by keeping the markers in place between the pre-treatment medical images acquisition and the treatment
- positioning the patient in for the treatment in a second position, in particular in a lying position (e.g. in Trendelenburg position)
- extracting information from the medical images and determining treatment parameters based at least on the information extracted from the medical images. This step can be performed either before positioning the patient in substantially lying position for treatment, or after, or even after positioning the patient in substantially lying position for treatment and positioning a treatment head onto the patient. For example, approximate or precise location of treatment slices along a vessel can be determined before treatment head positioning, or after treatment head positioning based on the positioning of the treatment head (e.g. every 3 mm starting from the position of the treatment head). Then, for each treatment slice, some parameters are preferably extracted from the medical images (preferably at least vessel diameter and/or depth under skin) and recommended treatment parameters are computed (e.g. by optimizing an objective function as per the current invention or using a rule, e.g. by dividing the local diameter by a constant value).

-    delivering a treatment on at least a vessel, preferably according to the recommended treatment parameters or before or during which the recommended treatment parameters are displayed to the user

In a further aspect of the invention, in the context of the treatment of one or a collection of vessels with HITU, a method may consist in:

-    collecting first imaging (preferably comprising at least B-mode images using an ultrasound hand-probe) of at least one of the vessels to be treated in a first position, preferably with the patient in substantially -standing position. In a preferred embodiment, a tracking,system (e.g. an optical tracking system) may track the position of the images (e.g. by tracking the ultrasound hand-probe). Preferably, the position of the images is known with respect to the patient: e.g. in the context of optical tracking, markers can be placed directly on the patient anatomy (e.g. using one or several disposable electro cardiograms patches or other skin-compatible adhesives) or on wearables (e.g. a kneepad) and their position and orientation is also captured by the tracking system. These markers are preferably substantially at the same position with respect to the target of the treatment during pre-treatment medical images collection and during the subsequent treatment. Preferably, this is achieved by placing the markers on one or several anatomical segments which do not move with respect to at least part of the target (e.g. on the thigh if the target is a vessel running in the thigh at least along a portion of its course) and by keeping the markers in place between the pre-treatment medical images acquisition and the treatment
-    positioning the patient in a second position for the treatment, preferably in a lying position (e.g. in Trendelenburg position)
-    delivering a treatment on at least a vessel
-    positioning the patient in a position for (immediate or remote) evaluation of the treatment outcome and acquiring second imaging data in the first position, preferably in a standing position.

In a preferred embodiment, e.g. in the context of a HITU treatment, the treatment software has a feature enabling to deliver a series of pulses or to treat a series of treatment sites.
First, the user places the treatment head at an appropriate position relatively to the target (preferably the center of the target, but alternatively to a side of the target for example). Alternatively, the device may automatically move to the appropriate position (e.g. if the position of the vein is known, e.g. by a manual interaction of the user with the device.
Then, using a dedicated control, the user may trigger the delivery of the sequence of pulses or the treatment of a series of treatment sites. Treating a treatment site can for example correspond to delivering a single pulse, or several pulses, or to deliver several pulses (e.g. with the same parameters, or with different parameters, e.g. with increasing power) until a certain effect is achieved (e.g. an HEM of a sufficient size occurs) on the treatment site.
Preferably, all pulses or treatment sites are in the same treatment slice. Preferably, the number of pulses or treatment sites in a treatment slice is between 1 and 10, and the spacing is between 0 and 10 mm. Preferably, the spacing and/or the number of treatment sites per treatment slice are limited such that all treatment sites are comprised in the live image (e.g. the number of treatment sites multiplied by the spacing is smaller than the image width), possibly with a margin (e.g. a fixed margin, or a margin which depends on the pulse type).
Preferably the location of the treatment sites (and/or a related information, such as the expected area of effect of the HITU beam associated with each and/or the collection of the treatment sites is represented at least when the sequence is triggered. Preferably, a robotic feedback loop (e.g. based on a registration or tracking algorithm) compensates for tissue dragging, thus ensuring that the geometrical spacing between pulses or treatment sites is respected. Preferably, the pulses are delivered, or the treatment sites are treated, one after the other without going back and forth (e.g. from left to right or from right to left). Alternatively, an alternate treatment order is implemented (e.g. the leftmost treatment site is treated, then the rightmost treatment site, then the leftmost of the remaining treatment sites etc.). Preferably, all pulses or treatment sites of a sequence within a treatment slice are delivered at substantially the same depth under skin (or at the same coordinate along the axis orthogonal to the skin). Preferably, the position along the axis orthogonal to the skin is manually set by the user, and only the lateral position of the pulses is handled by the feature. Preferably, all pulses are delivered with the treatment head or any other device (such as an inflatable balloon) exerting substantially the same pressure onto the skin. Preferably, at the end of the pulse sequence or if the pulse sequence is interrupted, the treatment head moves (preferably only laterally) to the appropriate position (e.g. the center of the target in the slice, or the position from which the delivery of the pulses was triggered). Preferably, all pulses are of the same type (frequency, duration). Preferably, the treatment sites are equally-spaced. Alternatively or additionally, the treatment head may also move to the next treatment slice (in the treatment progression direction, e.g. the next treatment slice distally or proximally to the current treatment slice).

Preferably, control unit of the treatment device implements an automatic power adaptation feature: if a pulse does not induce the desired tissular effect (e.g. pulses which does not induce HEM, or HEM of a sufficient size, or a sufficient temperature elevation) another pulse is automatically repeated at the same treatment site (optionally, with an increased power) before moving to the next predefined location or the treatment head moves to the next treatment site, but the power is increased. Preferably, additionally or alternatively, if a pulse induces a too big HEM, the series of pulses is automatically interrupted and/or the power of the next pulse is significantly reduced.

Preferably, in case of user action,the sequence is paused but the current status is kept in memory and the user can resume the sequence with a dedicated control. Alternatively, in case of user action, the sequence is cancelled. Alternatively or additionally,some user actions (such as changing the power) are allowed and do not cancel or pause the sequence, while other actions (e.g. manually displacing the treatment head) pause or cancel the sequence.

In a preferred embodiment, the sequence of treatment sites encompasses several treatment slices. Preferably, the treatment slices only encompass a small portion of the whole target (preferably < 20 % of the length or of the volume of a target). Preferably the number of treatment sites and their spacing is the same in all treatment slices comprised in the sequence.

The invention further provides a treatment device, preferably a HITU treatment device, which may have a feature enabling to lock and unlock rotation of a treatment head along a main ultrasound propagation axis of an embedded imaging probe. Locking is preferably implemented under the form of a mechanical blocking of the rotation (e.g. using electrobrakes or by disabling the possibility to trigger rotations with motors). Unlocking may either mean releasing a mechanical constraint (e.g. electrobrakes) or enabling the possibility to trigger rotations with motors). Alternatively or additionally, locking may mean setting an angle and storing its value (e.g. to define a treatment progression direction), and unlocking may mean exploring or enabling to explore different angles with the aim of redefining this angle. Preferably, unlocking is performed with a low pressure exerted on the tissue so as to avoid deforming the anatomy due to friction during the rotation and/or properly visualizing the target (e.g. a vessel is preferably not collapsed by the pressure). For example, if a HIFU device comprises an inflatable balloon and a way to control the pressure in the balloon, e.g. as described in EP 3 720 356, the pressure is preferably set to a low value (preferably < 40 mbar,most preferably < 15 mbar). It will be understood that the treatment head is particularly suitable to be used in any of the methods disclosed herein as well as in combination with other disclosed device aspects.

For example, a method of using such device may comprise the steps of:

- positioning a patient in treatment position
- positioning a HIFU treatment head such that the focal spot is in the vicinity of a target
- preferably locking (e.g. using electro-brakes) all degrees of freedom of the treatment head except the rotation around the main ultrasound axis of the embedded imaging probe
- rotating the HIFU treatment head, by a first angle, around the main ultrasound axis of the embedded imaging probe in order to obtain a longitudinal view of the target
- optionally rotating by a known angle (e.g. 90°) around the main ultrasound axis of the imaging probe
- performing the following steps in any order:

  o defining a treatment progression direction based on the value of the first angle , wherein the treatment progression direction may be parallel to the longitudinal axis of the target, at least in a plane orthogonal to the main ultrasound axis of the embedded imaging probe or of the HIFU transducer)
  ∘ optionally rotating by a known angle around the main ultrasound axis of the imaging probe,
  ∘ locking the rotation around the main ultrasound axis of the embedded imaging probe to avoid unwanted rotations (e.g. by touching the treatment head),
  ∘ preferably defining the locations of (preferably equally-spaced) treatment slices along the treatment progression direction.

Defining such a treatment progression direction may be especially advantageous in combination delivering a series of pulses or to treat a series of treatment sites.

Preferably, the treatment interface comprises at least a control to move between treatment slices, in particular in incremeents of +1 or -1, along the treatment progression .direction.

For example, if all treatment sites of a series are located in a treatment slice, the treatment head may come back to an initial position after the treatment of the series of treatment sites, i.e. after the slice has been treated. Moving to the next treatment slice along the treatment progression direction may place the treatment head in appropriate position to treat the new treatment slice by triggering the treatment of a new series of treatment sites.

After the treatment of a series of treatment sites, the treatment head may come back to the center of the vessel and, since the treatment progression direction is along the vessel, the treatment head may remain centered on the vein when moving to the next treatment slice. This may be particularly advantageous when the vein is insufficiently visible, rendering locating the vein e.g. difficult and/or time-consuming. Thus, the treatment device and methods of the invention may enable, in some aspects, the reduction of time required to locate vessels to be treated while still delivering pulses to the desired target.

In some treatments, however, the patient may move (e.g. due to pain) or the vein may not be straight along all its course. When the treatment progression direction is not aligned with the vessel anymore, the vessel may move in the image (e.g. if the treatment head angle in treatment is such that the vein is visible in transverse view) when the treatment head moves from one treatment slice another. Thus, it may be advantageous to be able to unlock the angle around the main ultrasound axis of the embedded imaging probe to define a new treatment progression direction during treatment. This may enable to keep the vein at the same position with respect to the image when moving from one treatment slice to another. Preferably the locations of the treatment slices are recomputed based on the new angle. Alternatively, only the treatment progression direction is modified.

In a preferred embodiment, the treatment interface enables to rotate the treatment head around the main ultrasound axis of the embedded imaging probe by 90°.

Preferably, at least part of the data used to define the model is obtained by the method of gathering treatment data and/or evaluating treatment data according to the invention.

An objective function may be defined based on the outcome metric. Preferably, it also incorporates other terms, for example to penalize treatment duration or the use of certain types of pulses which are more likely to induce perivenous tissue damages. In the following, a "value" of a treatment characteristic may be understood as a scalar value or a vector of values, preferably each corresponding to a segment of the body region or to a HITU pulse. Preferably, the value of the at least one treatment characteristic is chosen to obtain an optimum of the objective function. For example, several values or a range of values of the treatment characteristic resulting in near optimal values of the objective function may be proposed to the user which can select the value to implement. In the following such an algorithm selecting a value of at least a treatment characteristic is called a treatment optimizer.

Preferably, at least one of the at least one treatment characteristic is a variable feature or a monitoring feature or a combination thereof. For example, the treatment characteristic can be a variable feature such as the acoustic power of HIFU pulses or a binary value describing if local anesthesia is used, or a monitoring feature such as the area of a hyperechoic mark.

Preferably, among the at least one treatment characteristic for which a value is automatically selected by a treatment optimizer, at least one treatment characteristic can vary spatially (e.g. the frequency and duration of HITU pulses, the acoustic power). Most preferably, it can vary at a scale smaller or equal to the scale at which the outcome metric is computed. For example, if the outcome metric is the shrinkage of a vein in each of a series of treatment slices along a vein, the acoustic power can vary for each HITU pulse, which correspond to a smaller scale than the treatment slice since several HITU pulses can be delivered in a single treatment slice. The number of pulses per treatment slice would be at the same scale since it can be different in each treatment slice. By contrast, a binary value describing if local anesthesia is used may be considered at a higher scale (depending on the thickness of the treatment slices) since local anesthetic typically diffuses along the vein and is likely to impact several treatment slices.

In the case where the at least one recommended treatment characteristic comprises monitoring features, the use of the recommendation issued by the automatic selection algorithm may require a "trial an error" approach, as such a feature may present an a posteriori criterion.

For example, if the treatment optimizer finds that a certain hyperechoic mark area (optimal hyperechoic mark area) is desirable to achieve HITU treatment success, a power adaptation strategy can be adopted since hyperechoic mark area typically depends on the acoustic power. For example, the user may start delivering pulses at a low power, and increase the power, preferably while delivering pulses on the same position, until a pulse induces a HEM with an area greater or equal to the optimal area. If the HEM is bigger than the upper limit of the range, the user may decrease the power. Preferably, at least an overlay (for example, an ellipse substantially centered on the expected location of the HEM with the optimal area, and/or an ellipse substantially centered on the expected location of the HEM with the area above which the user is advised to decrease the power) may be displayed on the screen after the pulses preferably on the live B-mode image, preferably after each pulse, in order to guide the user in the adaptation of the treatment power. Then, the user continues to treat and continuously adapts the power after each HITU pulse to stay as close as possible to the optimal hyperechoic mark area, decreasing the power when the hyperechoic mark induced by a pulse is too big, and repeating a pulse at the current position if the hyperechoic mark is too small. Preferably, the treatment optimizer is designed to output ranges of optimal values for monitoring features instead of single values, due to the indirect control on these features.

The selection may optionally be based on a mathematical and/or statistical model and/or an artificial intelligence model and/or the output of an inferential statistical analysis-stored on a memory device.

Preferably optimizing the objective function is done in the sense which maximizes the desired clinical treatment outcome (for example maximizing the shrinkage or occlusion, and/or minimizing the perivenous tissue damages if applicable). For example, if the outcome metric is vein shrinkage, the objective function may also be the shrinkage of the vein, treatment characteristic may be the power of HIFU pulses and optimization consists in maximizing the shrinkage.

Although the outcome metric may be computed for at least two segments and thus depicts a local outcome, the objective function may be a scalar-valued function aggregating the result on all segments to a single score.

For example, where the outcome metric is the shrinkage of a vein, the objective function may be the mean shrinkage along the vein. This aggregation is particularly advantageous to include global penalizations (e.g. a penalization of the total duration of a HITU treatment) in the objective function. Typically, if each segment is considered separately, the penalization of the treatment duration on each segment may prevent proper treatment of some segments to avoid spending too much time. By contrast, if the objective function aggregates the whole treatment, it enables a treatment optimizer to consider spending a lot of time on a given segment if needed when some time can be saved on another segment, resulting in a reasonable total treatment duration.

The objective function may also be computed based on a model, for example a trained model. For example, the outcome metric may be the shrinkage of a vein, and the objective function may return the probability of occlusion of the whole vein, the probability of occlusion of the whole vein being computed based on the shrinkage profile along the vein using a model trained on clinical data.

The process of finding correlations between treatment characteristic and the outcome metric, or the training of a model to predict the outcome metric based on treatment characteristics may reveal that the features which can be extracted from the imaging data (e.g. local vein diameter, or depth under skin) are not strongly correlated with the value of the outcome metric. For example, vein shrinkage may only be correlated with the power used during a HITU treatment and patient's age. In this case, the process of determining at least one treatment parameter does not require to collect imaging data. Thus, preferably only the useful treatment characteristics which can be input to the model are captured beforehand (e.g. patient's age) and the model selects the appropriate value of the treatment parameter based on these data. Alternatively, first and second imaging data are still collected to compute the outcome metric in order to retrain or improve the model based on the outcome of the current treatment.

For example, a method according to the invention may consist in collecting first imaging data of a body region before a treatment and second imaging data of the body region after a treatment. Then, corresponding first and second reconstructed models are generated and at least an outcome metric is computed based on the reconstructed models. Preferably, other data are collected, preferably related to the patient (e.g. age, sex, weight, and/or medical history), the target of the treatment (e.g. depth under skin of the target) and/or the outcome of the treatment (occlusion of a vein, compressibility, and/or hyperechogenicity of tissues surrounding the target).

Then, a model, preferably an artificial intelligence model, is trained based on the collected data. For example, the model can be trained to predict the value of the outcome metric itself (e.g. the shrinkage of a vein in a series of locations along the vein) or another target feature which is preferably computed based on the outcome metric (e.g. the mean shrinkage along a vein, or the probability of occlusion of a vein). As known by the person skilled in the art, the model can result from a supervised learning. For example, it can be a regressor. Additionally or alternatively, the model can result from non-supervised learning.

Preferably the depth under skin of the target (with the patient in treatment position and/or in another position suitable for imaging data acquisition) is accounted for as an input feature of the model.

Finally, treatment parameters may be chosen by a treatment optimizer which optimizes an objective function. As known by the person skilled in the art, algorithms such as grid search can be used to automatically select an optimal value of at least one treatment parameter.

Alternatively or additionally, non-supervised learning can also be used, alone or combined with supervised learning. For example, a target feature can be defined by assigning each treatment slice to a class based on the outcome metric (e.g. the local shrinkage of a vein in different treatment slices along a vein). For example, the treatment slices are preferably assigned to the class "success" when shrinkage is above a certain threshold (preferably greater than 20 %) and/or the vein is closed after treatment. The treatment slices are preferably assigned to the class "failure" when shrinkage is below a certain threshold (preferably smaller than 50 %) and/or the vein not occluded after treatment. Other treatment slices may be discarded from the analysis, in particular if said treatment slices do not fulfill any of the criteria for success of failure. Shrinkage and vein occlusion may or may not be assessed after the same follow-up duration. For example, shrinkage may be assessed in acute and vein occlusion may be assess at a sub-chronic timepoint. Then, a (supervised) metric learning can be used to learn a metric which maximizes the distance between treatment slices assigned to the "success" class and treatment slices assigned to the "failure" class. Then, the features are preferably weighted by the coefficients resulting from the metric learning. Then, a non-supervised clustering algorithm is preferably used. Intrinsically, the resulting clusters are substantially homogeneous in terms of successes and failures.

Then, for a given treatment slice, for which a value of at least one treatment parameter is to be automatically selected, a subspace can be extracted from the normalized space based on the values of the fixed features of the given treatment slice (e.g. the subspace where the patient age, sex, body-mass index and the vein diameter correspond to the given treatment slice). Within this subspace, a cluster comprising mainly treatments slices assigned to the "success" class is selected (for example, the one with the higher mean shrinkage), and the value of the at least one treatment parameter is chosen so that the treatment slice falls as close as possible from the centroid of this cluster for example.

The invention is further directed to a method of treating a patient, in particular with HITU.

The method preferably comprises obtaining first imaging data, in particular a first model, of a body region to be treated. Preferably, a reconstructed model is created from the first imaging data as part of the method. Alternatively, the imaging data is formed by a reconstructed model which is obtained. Optionally, from the imaging data of the body region, at least two segments are defined. Optionally an outcome metric for the at least two segments is determined.

A value of at least one treatment parameter is selected, wherein the value is suitable for achieving an optimization of an objective function, i.e. for achieving an optimized value of an objection function. Preferably, the optimization of the objective function is performed taking into account at least two segments. Particularly preferably, the selection of the value is performed using a method of defining treatment parameters as disclosed herein. In particular, the objective function takes into account an outcome metric, preferably an outcome metric defined for the at least two segments.

In some embodiments, said value is selected from a range of values or set of values of the treatment characteristic.

The body region is treated using the determined treatment characteristic. Preferably, the treatment is conducted by impinging the body region with a HITU beam.

Preferably, the objective function is at least partially a function of a difference in the body region before and after (or during) the treatment, in particular for given values of fixed features.

It will be understood that the outcome metric may represent the difference in the body region.

Preferably, the method further comprises the steps of obtaining second imaging data after treating the body region. A second, preferably three-dimensional, reconstructed model may be created from the second imaging data. The first and second reconstructed models, both of which are preferably three-dimensional and/or reconstructed volumes, are registered. Based on the registered first and second reconstructed model, the achieved outcome metric can be determined. In particular, the outcome metric may be determined for at least one or at least two segments of the model. The outcome metric is preferably the same outcome metric which was determined prior to the treatment as the outcome metric to be achieved. However, it will be understood that any other outcome metric as defined herein may also be determined. The value of the treatment characteristics, the outcome metrics, and preferably the imaging data are stored in a memory.

In particular, the above-described method steps are one preferred way of obtaining the first and second imaging data for any other method described herein.

The invention is further directed to a method of treating a patient wherein a depth under a skin is estimated based on fixed features and/or first imaging data. Particularly preferably, the depth under the skin is estimated based on an outcome metric representing a change before and during a treatment as described herein above. A treatment parameter is selected which is suitable with the estimated depth under the skin.

For example, the outcome metric may be a distance between a patient's skin and a tissue region to be treated, for example a vessel, i.e. a tissue depth, under treatment conditions (e.g. with compression from a treatment device). The treatment parameters may be chosen such as to not burn skin and/or deliver a suitable thermal dose to the vein, treatment parameters may for example be the necessity of injecting fluid, a pulse type, or even the possibility to perform a HITU treatment with a good chance of achieving a clinical success. In the latter case, the treatment optimizer may thus be considered a patient selection software.

The invention is further directed to a computer program product, in particular a software, comprising instructions which, when the program is executed on a computer, cause the computer to carry out the steps of any of the methods described herein.

In some embodiments, a software may be provided which allows a user to input patient data and get treatment recommendations in return based thereon. In particular, such a software may be used to perform any method disclosed herein or may be part of a system according to the invention.

Preferably, such software is adapted to predict clinical success on the scale of the structure of interest. For example, another machine learning algorithm may be trained to predict the clinical success (at the scale of the structure of interest) based on an outcome metric and/or based on the treatment parameters. Once trained, this algorithm may be used to recommend treatment characteristics.

Preferably, the system and the methods according to the invention are used in the treatment of veins.

Preferably, a first set of acquired data is collected during an ultrasound acquisition, preferably performed with a hand-probe.

A second acquisition of the same structure of interest at substantially the same location may then be performed at a different time. Preferably, the same imaging device/acquisition system is used (for example a hand-probe). Thus, the registration of the two acquired data may be performed after the second acquisition. For example, the two volumes may be reconstructed and parallel planes may be displayed, preferably in at least two different figures. Alternatively or additionally, the acquired image sequences are displayed. Then, at least a control, preferably at least a slider, may be used to move within the volume in a spatially synchronized manner. At least a second control, preferably a slider or a pair of switches, enables to change a spatial offset between the two volumes to manually register them.

In one preferred embodiment, imaging data which is suitable to be used with any method or system described herein is obtained as described in the following. In this example, a great saphenous vein (GSV) is imaged, but it will be understood that any other structure may be evaluated as well.

A B-mode acquisition along the portion of interest of the GSV is performed, with the patient preferably in standing position.

Compressibility and/or flow in the GSV may then be assessed by the user, optionally through a separate scan, with the patient preferably in standing position.

The reconstructed vein may be displayed. The 3D conformation of the vein is not necessarily preserved by the representation, but instead only the vein radius is plotted with respect to the abscissa along the leg or with respect to a curvilinear abscissa along the vein, as this information may be sufficient for the user in some instance. It will be understood that the conformation of the vein may also be evaluated, where desired.

Compressibility and/or flow information may be automatically extracted from the acquired data. Additionally or alternatively, the user may use an interface to extract useful images or videos or to review the extracted data of the automatic algorithms.

Using health data to predict the success metric can lead to patient selection guidelines. If information about the structure of interest are also considered (for example, pre-treatment target size), this can also lead to the development of patient selection guidelines or to a software which automatically computes the chances of success of a therapy based on a first set of acquired data.

For example, the dataset can comprise a set of treatment slices, preferably of different patients, with, wherein for each slice the number of delivered pulses, the mean power used over all delivered pulses, the mean HEM area over all delivered pulses, and/or the mean depth under skin of the acoustic focus are contained in the dataset. It will be understood that further variables disclosed herein may be used.

For example, the outcome metric may be the shrinkage of a vein or the coagulated area for a tumor.

For a tumor, the coagulated area corresponding to a treatment slice can for example be computed based on an elastography acquisition or based on the injection of an ultrasound contrast agent and a B-mode scan.

Recommendations may be given in the Instructions For Use (IFU) of the device or during training.

Alternatively, the outputs of the machine learning algorithms can serve to define graphical elements which help the user to deliver the optimal treatment.

For personalized treatment recommendations, input features preferably include variable features and/or monitoring features.

The target is preferably an outcome metric. For general treatment recommendations, the outcome metric is preferably at the scale of the treatment slice.

For example, a dataset may comprise a set of treatment slices, preferably of different patients, wherein for each treatment slice the age of the patient, the BMI of the patient, the mean pre-treatment diameter of a vein or pre-treatment volume of a tumor, the number of pulses delivered, the mean power used over all pulses delivered, and/or the mean HEM area over all pulses delivered is comprised in the dataset.

For example, the target may be the shrinkage of a vein or the coagulated area for a tumor.

For localized treatment recommendations, input features preferably include fixed features, variable features and/or monitoring features.

The target is preferably an outcome metric. For general treatment recommendations, the outcome metric is preferably at the scale of the treatment slice.

For example, the dataset may comprise a set of treatment slices, preferably of different patients, wherein for each treatment slice the age of the patient, the BMI of the patient, the pre-treatment diameter of a vein /pre-treatment area of a tumor, the depth under skin of the vein, the number of pulses delivered, the mean power used over all pulses delivered, and/or the mean HEM area over all pulses delivered are contained in the dataset.

For example, the target may be the shrinkage of a vein or the coagulated area for a tumor.

Compared to personal or general treatment recommendations, localized treatment recommendations typically includes data wherein the pre-treatment diameter and the depth under skin of the vein is known for each treatment slice.

The invention is further directed to an interface which may be used with a system according to the invention. The treatment interface may in particular be adapted to display recommendations computed before treatment. Furthermore, the parameters of the next pulse to be delivered may be displayed. The interface may further comprise an input means with which a user may validate and/or correct recommended parameters. The user may also request further explanations using a dedicated control. Information may be displayed as known in the art. For example, for image-based decisions using neural networks, heat maps may be overlaid on an image shown on the interface, thus for example showing the relative weight of pixels using back-propagation techniques. If several features are used for the decision (e.g. patient characteristics, medical images, etc.), a dedicated figure may show the relative weights of the different features which motivated the decision. The explanations may result from the choice of explainable machine learning techniques adapted to not only output a result, but also explanations (preferably in the form of graphical representations, such as heatmaps, or in the form of a list of the most important features responsible for the output, preferably complemented with the relative weights of the features) .

The treatment recommendations may vary over time and be influenced, for example, by previous pulses parameters and/or an effect of the pulse visible on the images (e.g. HEM and/or hyperechogenicity of the surrounding tissues). These recommendations preferably include technical parameters (e.g. power, location of the pulses, tilt of the treatment head, and/or pressure exerted onto the tissue) and preferably also general recommendations.

For example, during a HITU treatment, the acoustic power is preferably limited based on the impinged skin area. In this context, the system may detect that the maximum power was reached without inducing HEM, thus that it may be necessary to ask the user to perform and injection to increase the depth under skin of the vein. Preferably, the user can interact with the system to input additional information (e.g. patient pain or discomfort) which are preferably accounted for by the recommendation algorithm.

The treatment may be fully automated or be a hybrid decision-support system.

Preferably, a general treatment strategy is defined prior to treatment either by a physician based on experience or based on treatment recommendations issued by an algorithm. For example, only the center or the periphery of a tumor may have to be treated. By contrast, in the case of a vein, only the tributaries, or a proximal part, or only a distal part may have to be treated by HITU, while other parts (in particular a distal part) may be treated by sclerotherapy.

A physician is not necessarily present during the whole treatment, and at least part of the treatment procedure may be performed by a non-physician. In particular delivery of pulses need not be performed by a physician.

Preferably, a range is defined for at least one treatment characteristic (e.g. for the number of pulses inducing HEM for each treatment slice), which are preferably validated by a physician. Validation may occur before treatment or after a treatment of at least one treatment slice, preferably before 20 % of the treatment slices are treated.

Preferably, the user can interact with the system to confirm and/or inform the system that he disagrees with the proposed decision. This information can then be used to improve the recommendations using reinforcement learning techniques, i.e. by training of machine learning algorithms. In particular, a data set may be marked an unsuccessful treatment and saved in a memory.

As a further example of AI algorithm, a logistic regression may be used. In particular, when considering an outcome metric at the scale of the treatment slice, a threshold on the outcome metric may be used to define the success. A logistic regression classifier may be trained to predict the success or failure in each treatment slice and to estimate the probability of success.

In particular, the following variables may be defined:

$p\_t$: probability for the current slice at time t to be treated successfully.
$X\_{(i,s)}$: features which cannot be controlled (for example, the fixed features)
$x\_{(i,a)}$: features which are controllable (for example, the variable features)
$N\_f$: is the total number of features
K: is the number of features which cannot be controlled
B: the parameters of the trained model

Then the following equation can be written:

$$\log\left(\frac{p_t}{1-p_t}\right) = \sum_{i=1}^{k} \beta_{i,s}\, x_{i,s}(t) + \sum_{i=k+1}^{N_f} \beta_{i,a}\, x_{i,a}(t)$$

Which can be rewritten as

$$\log\left(\frac{p_t}{1 - p_t}\right) = C(t) + \sum_{i=k+1}^{N_f} \beta_{i,a}\, x_{i,a}(t)$$

where $C(t) = \sum_{i=1}^{k} \beta_{i,s}\, x_{i,s}(t)$

$$C(t) = \sum_{i=1}^{k} \beta_{i,s}\, x_{i,s}(t)$$

The above equations represent a linear optimization with constraints (for example acceptable parameter ranges.) as known by the skilled person.

Other algorithms may also be implemented, in particular for treatment automation. For example, in the context of the thermal occlusion of veins, a vein tracking algorithm can be implemented. This is especially advantageous in combination with a position sensor which may give a precise position estimate along the vein while accuracy in a transverse plane may be more challenging, in particular due to pressure on the tissue, tissue dragging by the treatment head and/or injections. In this case, the position of the treatment head along the vein is known according to the position sensor but the lateral and vertical position in the planes orthogonal to the vein may be determined by the tracking algorithm. In particular, the tracking algorithm may be used in a feedback loop when moving a robot, to compensate for tissue dragging.

For tumors, tracking algorithms may present certain advantages as well. It will be understood that a tracking algorithm is not necessary, however. A user may place the treatment head in a given position (for example, such that the focus is in the center of a nodule), and the treatment can be executed by estimating the localization of the focus by integration of motions. An expected contour of the tumor and/or other landmarks may be displayed to allow the user to verify that the current location of the treatment head is consistent with the expected location.

The invention is further directed to system for gathering treatment data and/or generating a treatment plan for treating a patient with HITU..The system comprises a computer device which can be brought in operative connection with a memory. The computer device is adapted to receive, via an interface, first imaging data of a body region and second imaging data of the body region. The system is preferably further adapted to create a reconstructed first model of the body region before a treatment and a second model of the body region after treatment has been conducted based on the first and second imaging data. Additionally or alternatively, the system is preferably adapted to receive a reconstructed first model of the body region before a treatment and a second model of the body region after treatment forming first and second imaging data. The system, preferably the computer device, is further adapted to, based on the first and/or the second imaging data, preferably based on the first and/or second reconstructed models, generate a visualization of a region to be treated before and/or after treatment, on a display. Preferably, the visualization is such that a change induced by the treatment is visible. A changed region may be high-lighted, for example.

The invention is further directed to system for gathering treatment data and/or generating a treatment plan for treating a patient with HITU. The system comprises a computer device which can be brought in operative connection with a memory. The computer device is adapted to receive, via an interface, imaging data of a body region. The system is preferably further adapted to create a reconstructed first model of the body region a after treatment has been conducted based on the imaging data. Additionally or alternatively, the system is preferably adapted to receive a reconstructed model of the body region after treatment forming imaging data. The system, preferably the computer device, is further adapted to, based on the reconstructed model, generate an outcome metric representing a difference between a lesion created by a pulse and a lesion expected to be created by said pulse. To this effect, the system may be adapted to compute an expected lesion from the used treatment characteristics.

The invention further relates to a training dataset for use in a method of training a machine-learning model. The dataset comprises first and second imaging data of a-body region of a patient. The first imaging data corresponds to a state before a treatment and the second imaging data corresponds to the body region after a treatment has been conducted. The first and second imaging data are registered. The training dataset may in particular be obtained by the method of gathering treatment data and/or evaluating treatment data according to the invention.

For example, the imaging data (or, in the cases where the imaging data are acquired data or reconstructed models, the first imaging data after some adequate processing was performed) may be a list of diameters along the vein, preferably at the position of the treatment slices, preferably complemented with depth under skin information (preferably at least pre-treatment), perivenous tissue damage area (post-treatment), and treatment characteristics/parameters used in the treatment slices. Typically, as known by the person skilled in the art, this can take the form of a table with each row containing the information related to one treatment slice.

The invention further relates to a system for treating a patient. The system may in particular comprise a data processing apparatus comprising means for carrying out any of the methods disclosed herein. The system comprises a memory comprising a mathematical and/or statistical model and/or an artificial intelligence model and/or the output of an inferential statistical analysis obtained by training a machine-learning algorithm with the training dataset according to the invention. The model may be adapted to be used by an optimizer to output at least a treatment parameter based on imaging data and/or treatment characteristics (e.g. value of a fixed feature) input.

For example, the system may comprise a HIFU device to treat vessels, in particular varicose veins. The HIFU device may comprise or may be in connection with a memory in which a model is stored. The model may have been trained to predict the value of an outcome metric (e.g. vessel shrinkage) or a related value based on first and second imaging data (e.g. B-mode scans of vessels) related to a treatment delivered with a similar HIFU device or with treatment characteristics compatible with the HIFU device of the system (e.g. similar frequencies, pulses durations, energies, and/or powers). The HIFU device may comprise or may be in connection with a means for a user to input treatment characteristics (e.g. patient data or first imaging data). The HIFU device may comprise or may be in connection with a computer (or any other suitable calculation device) able to run an optimizer which, using the model, may output at least a treatment parameter (e.g. number of treatment sites per slice) corresponding to a suitable (e.g. optimal) value of the objective function used by the optimizer and based on the outcome metric.

The invention will now be explained in more detail with reference to the following figures, showing:

Figs. 1a-1e:    schematically different embodiments of a system.
Figs. 2a-2c:    schematically imaging data being reconstructed into a model.
Figs. 3a-3d:    schematically the determination of an outcome metric from segments.
Figs. 4a-4d:    schematically the determination of a different outcome metric from segments.
Figs. 5a-5b:    schematically different treatment devices.
Fig. 6:         schematically a data set.
Figs. 7a-7b:    schematically the use of a sensor as a landmark.
Fig. 8:         schematically a manual registration with an interface.
Fig. 9:         schematically an interface with a 3D view of a vessel.
Fig. 10:        a further embodiment of an interface.
Fig. 11:        a further embodiment of an interface.
Fig. 12:        an example of an evaluation using a HEM.
Fig. 13:        an example of a heat map.
Fig. 14:        a flowchart illustrating treatment success.
Fig. 15:        a further embodiment of an interface.
Fig. 16:        a further embodiment of an interface.
Fig. 17:        a flowchart of a patient management process.
Fig. 18:        a further embodiment of an interface.
Fig. 19:        schematically the acquisition of B-mode images.
Fig. 20:        schematically a treatment of a tumor.
Fig. 21:        schematically a representation of veins at different points in time.
Fig. 22:        a schematic representation of treatment characteristics.
Fig. 23:        schematically a method for training a model.
Fig. 24:        schematically a method of using a trained model.
Fig. 25:        schematically a method of selecting a treatment characteristic.
Fig. 26:        a further embodiment of an interface.
Fig. 27:        a schematic representation of a segmented vessel.
Fig. 28:        schematic of a HIFU treatment interface.
Fig. 29:        schematic illustration of a treatment progression.

An anatomical structure of interest may be understood as an anatomical structure (e.g. an organ, a vein, or a tumor) or group of anatomical structures, preferably comprising a pathological structure (such as an incompetent vein or a tumor) or an anatomical structure to be diagnosed.

A treatment slice may be understood as a 2D plane comprising an anatomical structure to be treated, in particular in the context of an ultrasound therapy and High-Intensity Therapeutic Ultrasound (HITU) therapy. Preferably, in particular for HITU therapy of truncal veins, treatment slices are substantially orthogonal to the longitudinal axis of the vein. Preferably, one or several ultrasound pulses can be delivered in one treatment slice.

A treatment slice may correspond to a segment.

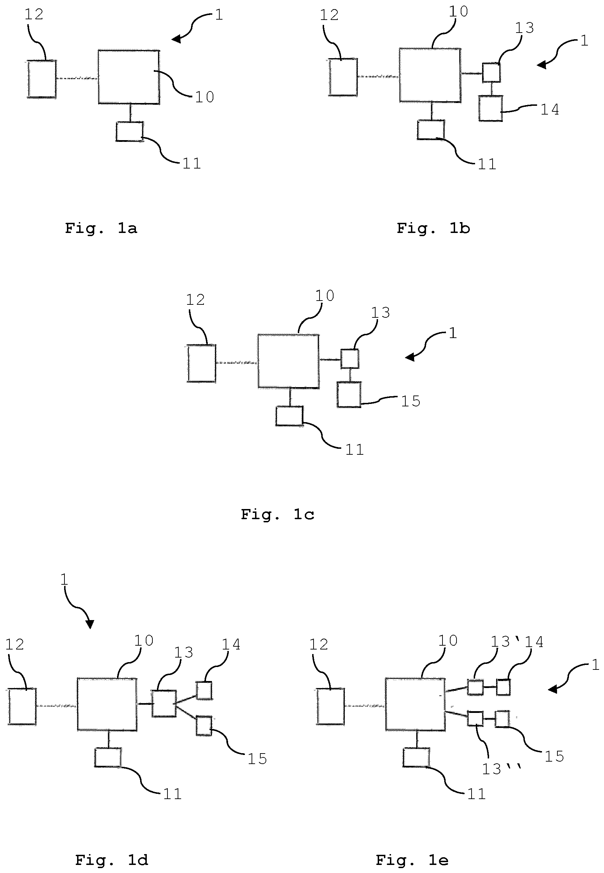

Fig. 1a schematically shows a system 1 according to the invention. The system comprises a computer device 10 with an interface 11. The interface 11 is adapted to receive data which may be processed by the computer 10. Any data may be transferred, however, the interface is adapted for transferring.imaging data. To this end, the interface is suitable for transferring file sizes typical for imaging data and thus provides a sufficient data transmission speed. It will be understood that any interface known in the art is suitable, however, a USB port is preferably used as an interface. Data may thus be transferred via a USB drive. Alternatively or additionally, the interface is a network connection enabling to receive data securely from one or several medical devices. The computer 10 is further associated and in operable connection with a memory 12. Any memory known in the art is suitable, for example a hard drive or an SSD. It will be understood that the memory 12 may be a part of or built into the computer 10. Preferably, however, the memory 12 is a separate unit which is only connected to the computer 10 via data transmission means such as a cable, a wireless connection, and/or the internet.

Fig. 1b shows a system 1 which is based on the system of Fig. 1a. In addition, the system shown here comprises a control unit 13. The control unit 13 is connected to a treatment device 14 and is adapted for controlling the treatment device 14.

Fig. 1c shows a system 1 which is based on the system of Fig. 1a. In addition, the system shown here comprises a control unit 13. The control unit 13 is connected to an imaging device 15 and is adapted for controlling the imaging device 15.

Fig. 1d shows an alternative embodiment of a system 1 which is based on the system 1 of Fig. 1a. The system1 comprises, in addition, a control unit 13 is connected to both a treatment device 14 and an imaging device 15, which are both controlled by the control unit 13.

Fig. 1e shows an alternative embodiment of a system 1, based on the embodiment of Fig. 1a, comprising a first control unit 13' and a second control unit 13'' . The first control unit 13' is connected to and controls a treatment device. The second control unit 13" is connected to and controls an imaging device 15.

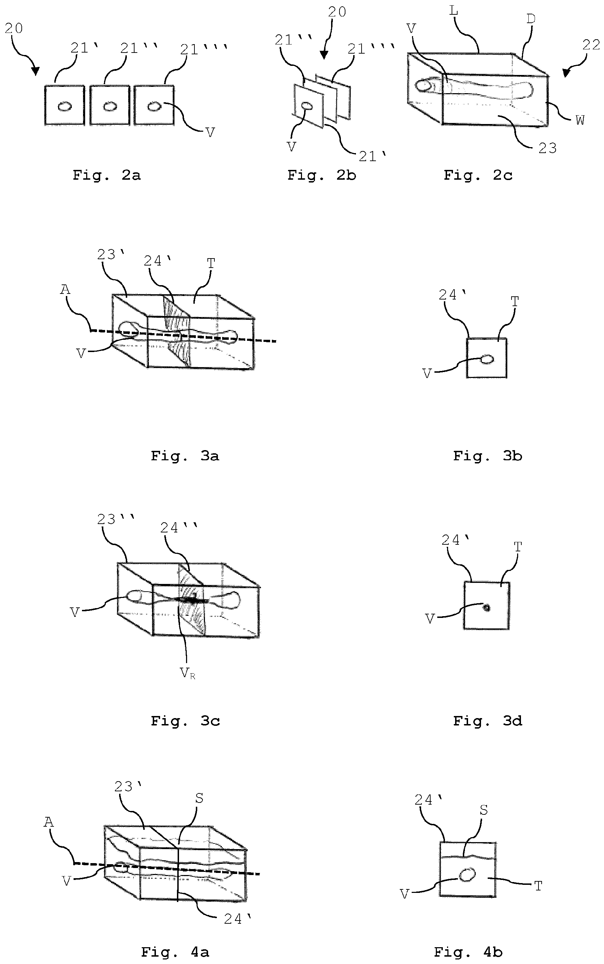

Fig. 2a schematically shows imaging data 20. Here, the imaging data 20 comprises three images 21', 21", 21''' taken at different positions along a longitudinal axis of a vessel V. Accordingly, each image 21', 21", 21''' shows a cross-section of said vessel V at a different position. The images 21', 21", 21''' are preferably substantially parallel, but they may not be strictly parallel (e.g. if they were acquired based on a manual US scan performed with a hand-probe).

Fig. 2b shows schematically an arrangement of the images 21', 21", 21''' parallel to one another at the distances corresponding to the distances between the positions at which the images were taken, i.e. forming acquired data. Between the images 21', 21", 21''', i.e. at positions where no images where taken, no data is available. Thus, certain characteristics, for example tissue types, vein diameter, or other parameter typically available from imaging data 20, is not immediately known for these positions. Thus, a model (see Fig. 2c) can be formed from the imaging data 20. The model is formed by interpolation of the images 21', 21", 21''' and estimation of changes in the tissue in between the images 21', 21", 21'''. It will be understood that the shown arrangement of images 21', 21", 21''' in a parallel manner is illustrative to conceptually describe the invention. In particular where a model is generated by a computer, it is not necessary to actually arrange images in this manner.

Fig. 2c shows a reconstructed model 22 formed as described in the context of Fig. 2b. The model 22 represents a reconstructed three-dimensional volume 23. Here, the volume 23 has a cuboid shape wherein the width W and depth D substantially correspond to the side length of the images 21', 21", 21''' and the length L corresponds to the total distance along which the images 21', 21", 21''' were taken. The vessel V is located inside the volume. Based on the model 22, it is possible to generate segments within the volume 23 (see Figs. 3a and 3b). Such segments substantially simulate imaging data taken as such a position and orientation.

Figs. 3a and 3c show reconstructed volumes 23', 23'' which have been created as described in the context of Figs. 2a-2c. The volumes 23', 23" represent a body portion of a patient with a vessel V surrounded by tissue T.

Fig. 3a shows a first volume 23' which was reconstructed based on first imaging data (not shown) of the body region. The first imaging data was collected before a treatment. Accordingly, the vessel V substantially corresponds, in particular in its size and shape, to the patient's anatomical vessel. As described above with respect to Fig. 2c, a segment 24' is created. The segment 24' can be created for any location or orientation within the volume 23'. Here, a plane substantially parallel to a longitudinal axis A of the vessel V is chosen which is located at a desired treatment location. Accordingly, the segment 24' shows a cross-section of the vessel V which allows to determine a geometry and a size of the vessel V, for example.

Fig. 3b shows the segment 23'. The vessel V is visible in a plane perpendicular to the longitudinal axis A. An internal and/or an external diameter of the vessel can be determined based on the segment 23'.

Fig. 3c shows a second volume 23'' which represents the same body region as the first volume 23' shown in Fig. 3a. However, the second volume 23'' was reconstructed based on imaging data (no shown) acquired after a treatment of the vessel V with HITU. Accordingly, the vessel V is collapsed in a region $V_R$ along the longitudinal axis A. A second segment 24'' is created based on the second volume 23''. The second segment 24'' is created at the same location as the first segment 24' (see Fig. 3a). It will be understood that the registration of the imaging data and/or the first and second volumes 23', 23'' can be done to ensure that the first and second segments 24', 24' are created at the same location. Registration is known in the art.

Fig. 3d shows the segment 24" in the same perspective as the first segment 24' is shown in Fig. 3b. As the second segment 24" is located at a treatment location where the vessel V is collapsed, the cross section of the vessel V in the second segment shows a reduced diameter which can be determined based on the second segment 24".

Comparison of the first and second segments 24',24" allows to determine an outcome metric. Here, the ratio between the diameter of vessel V in the first and second segments 24', 24" is chosen as the outcome metric and is approximately 0.5, meaning the diameter of the vessel V is halved after the treatment compared to before the treatment. It will be understood that any other outcome metric may be chosen which represents a change in the vessel properties due to the treatment.

Figs. 4a-4d show a first and a second volume 23', 23" similar to Figs. 3a-3d. Here, a different success metric is determined.

Fig. 4a shows a tissue T which is delimited by a skin surface S. A vessel V is located underneath the skin S in the volume 23'. A segment 24' (only outlined for clarity) is chosen within the volume and in a plane substantially perpendicular to the longitudinal axis A of the vessel V.

Fig. 4b shows the segment 24' with the vessel V in the tissue and the skin surface S above. The vessel V has a round cross-section.

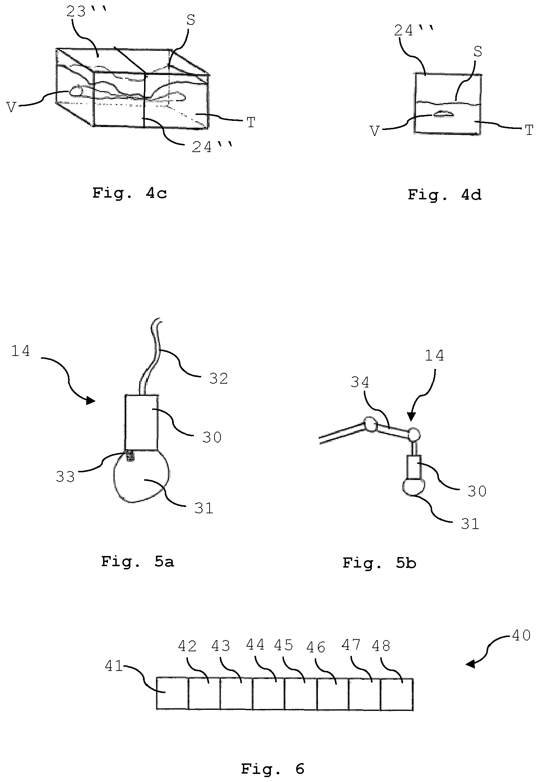

Fig. 4c shows a second volume 23" based on imaging data (not shown) acquired during a treatment. The treatment (not visible in the volume 23", which is a reconstructed model) was conducted with a HITU treatment head (see Figs. 6a and 6b). During the treatment, the treatment head exerted a force on the skin and compressed the tissue T and the vessel V therein.

Accordingly, as can be seen in Fig. 4d, the vessel V is compressed into a substantially flat shape. A distance between the skin surface S and the vessel V is reduced.

As described above, comparison of the first and second segments 24', 24" allows to determine an outcome metric. Here, the change in distance between the vessel V and the skin surface S between the first and second segments 24', 24" is chosen as the outcome metric. Here, the distance has changed by 5 mm to a total distance of 3 mm in the segment 24", i.e. during the treatment. It will be understood that any other outcome metric may be chosen in the shown case, i.e. where the second volume 23" corresponds to a state during a treatment.

It will be understood that in the concepts shown in Figs. 3a-4d, any number of segments can be evaluated to determine an outcome metric. For clarity, only one segment is shown and described in the above figures, however, two or any other number of segments in the volumes may have been selected and the outcome metric determined.

Fig. 5a shows schematically a HITU treatment head 14 which may be used as a treatment device in any of the systems according to the inventions (see Figs. 1a-1e). The HITU treatment head 14 comprises a handle 30 and a balloon 31 attached thereto. Here, the treatment head 14 also comprises a pressure sensor 33. The pressure sensor is adapted to measure a pressure inside the balloon 31. During a treatment, the pressure inside the balloon correlates with the pressure exerted on the patient's skin. The pressure sensor 33 may be in operable connection with a computer which is part of the system according to the invention. It will be understood that a pressure sensor 33 is not necessary for a treatment head 14 to be suitable for a system according to the invention. The treatment head 14 comprises a cable 32 which allows for data transfer.

Fig. 5b shows a treatment head 14 with a robotic arm 34. Here, the treatment head comprises a handle 30 and a balloon 31. The treatment head 14 may in particular be a treatment head as shown in Fig. 5a, or any other treatment head known in the art. The robotic arm 34 may exert a pressure on a patient's skin by pushing the treatment head 14 toward the patient's skin.

Fig. 6 schematically shows a table containing data which may be stored in a memory of a system according to the invention. Here, the table 40, which represents a data set from one treatment, comprises 8 data fields 41, 42, 43, 44, 45, 46, 47, 48. A data set 40 contains at least one value of an outcome metric, though typically at least two values of an outcome metric respectively corresponding to at least two segments are determined. For example, any outcome metric as described above may be used and stored. Further data units may be patient parameters, such as sex, BMI, and age, and/or treatment characteristics such as number of pulses, pulse power, pulse duration.

It will be understood that data sets 40 such as the one shown here are collected in some methods according to the invention. The system according to the invention typically has a plurality of such data sets 40 stored in the memory in order to determine treatment characteristics based on a pre-determined outcome metric. Accordingly, in a method of treating a patient according to the invention, a plurality of data sets 40 as schematically shown here may be used to determine which treatment characteristics may lead to a certain outcome metric. It will be understood that the data may be multimodal data, thus all data fields 41, 42, 43, 44, 45, 46, 47, 48 are not necessarily of the same nature (e.g. medical images, tabular data, and/or text).

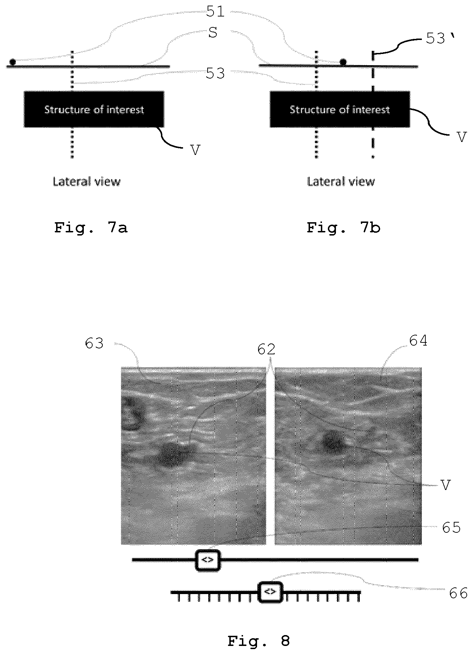

Figs. 7a and 7b show different illustrations of a use of a unique landmark, here a sensor 51.

On the registration of a single imaging plane under the hypotheses that a sensor provides the position of the current imaging plane 53 with respect to the landmark 51 and no other information is necessarily extracted from the geometry of the structure of interest or its surroundings.

As shown in Fig. 7a, the sensor 51 is located in the vicinity of a structure of interest, here a vessel V. A distance between the sensor 51, which acts as a landmark, determines a position of the imaging plane 53 along the structure.

As shown in Fig. 7b, where a landmark, here a sensor 51, is placed on a vessel V which is to be imaged, it may not be possible, based on the distance between the sensor 51 and the imaging plan 53 to unambiguously determine a location along the vessel V. For illustration, a second imaging plane 53' is shown, which is located at a different position but which has the same distance to the sensor 51. It will be understood that a marker may be used instead of a sensor.

However, even a positioning of a landmark as shown in Fig. 7b may be employed. In particular where 3D registration of the acquired data is performed after substantially all the volume has been acquired, it is possible to resolve the directional ambiguity shown in Fig. 7b by comparing the extent (i.e. length) of the 3D structure on each side of the landmark. Additionally or alternatively, an orientation of the acquisition can be standardized (e.g. from the proximal to the distal part of the leg).

It is also possible, however, to use other information (for example, the position of other landmarks) in order to register the acquired data.

Fig. 8 shows an embodiment of an interface suitable for manual registration of two acquired data which were reconstructed. In this example, a first image 63 corresponding to a first volume (not shown) and a second image 64 corresponding to a second volume (not shown) are visible. The first and second images 63, 64 do not correspond to the same location in the body region based on which the first and second volume were reconstructed, as can be determined because a tributary 62 is closer to the vein V in the first image 63 than in the second image 64. Accordingly, an offset can be manually adapted using a slider 66 to bring the first and second images 63, 64 in accordance.

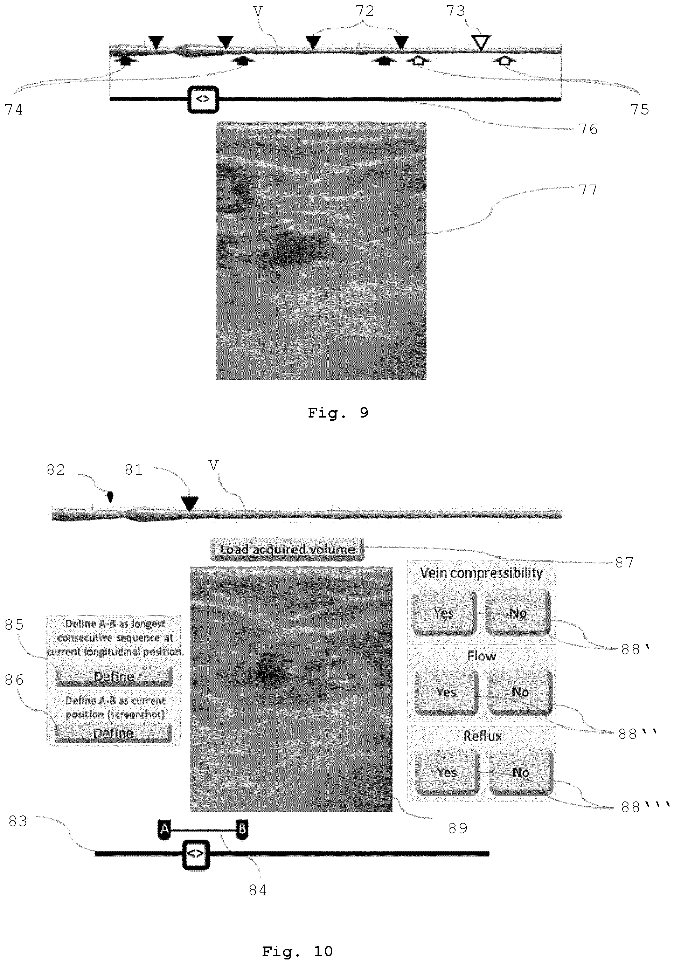

Fig. 9 shows schematically a 3D representation of a vein V. Here, only the evolution of the diameter of the vein V along an axis is represented. By contrast, the 3D conformation of the vein V (i.e. its 3D trajectory in the anatomy) is not shown in the present example. It will be understood that the conformation may also be determined where necessary or desired. However, here a radius is simply plotted with respect to an abscissa along the leg.

Markers 72, 73 represent different points along the vein V where a compression was applied during imaging before after a treatment. It will be understood that a similar representation may also be done with imaging data from before or during a treatment. Markers 72 are adapted such that a user can differentiate them from markers 73, here by black and white colors. Markers 72 show positions where the vein V is incompressible. Markers 73 show where the vein V is compressible. It will be understood that any other differentiation (shape, color, orientation, etc.) of the markers 72, 73 may be used, and that it would also be possible to use a color scale to quantify compressibility. Arrows 74, 75 represent different points along the vein where flow was assessed using Doppler. Arrows 74 are colored in black and indicate where no flow is visible (i.e. where the vein V is occluded). and/or where no reflux is visible whereas arrows 75, which are colored white, indicate where flow is visible. As described above, it would also be possible to adapt the represensation of arrows 74,75 in other ways, or it would be possible to use a color scale to quantify doppler flow (speed, reflux duration and/or percentage of the lumen in which flow is measurable for example). It would also be possible to highlight continuous zones in which the vein is compressible, or incompressible, or in which there is flow, or no flow, according to the captured information. In this case, the zones between a point where the vein is compressible and a point where the vein is incompressible is preferably not attributed to any of the two classes (compressible/incompressible), and similarly for the flow information.

Here, the interface also comprises a slider 76 to navigate within the acquired data or within a reconstructed model to display an image or a segment 77 within the volume, which may be an acquired or a reconstructed image 77.

In this example, compressibility is assessed using automatic image processing algorithms which are used to segment the vein V as it is collapsed by the user, but compressibility could also be manually indicated by the user (either in real time with a dedicated control, or a posteriori, when reviewing the acquired video sequence).

The detection of the occurrence of these compression sequences can either be automatic (using the position sensor of the hand probe and/or a force sensor and/or image processing algorithms e.g. AI algorithms) or these sequences can be indicated by the user (either during the scan using a dedicated control such as a button or a posteriori when reviewing the acquisition). Additionally or alternatively, a whole scan of the vein V can be performed while continuously applying a high compression.

It will be understood that flow as measured by Doppler mode may be continuously visible or may also be visible only during specific sequences (e.g. caused by tip toeing, Valsalva maneuver, distal compression of the leg by the practitioner) Preferably, the maximum flow which is captured for a given position is shown on the.interface. It is also possible to manually enter whether a flow is visible at a given position, e.g. by a user conducting the measurements (either during the acquisition or a posteriori) .

A presence of a reflux may also be detected as a temporary inversion of the flow direction and indicated on the interface.

Preferably, compressibility and/or flow information are not only displayed at specific points but may be interpolated. For example incompressible segments can be represented between points where the vein was assessed as incompressible.

The representation shown in Fig. 9 may be used to assess the outcome of a thermal ablation, in particular since the shown representation makes the comparison between the vein V at several time points easier. Abscissa could also be a curvilinear abscissa along the vein, or the 3D conformation of the vein can be represented. The diameter was computed using a segmentation algorithm in parallel slices of a reconstructed volume. Preferably, the diameter is computed as the maximum diameter recorded for a given spatial abscissa. Preferably, the diameter designates the outer-diameter of the vein V (with respect to the adventitia) or the inner diameter of the vein (with respect to the intima). Other anatomical information can also be represented (tributaries, junction with deep system, escape point, or others).

In general, it is possible to perform individual scans for each purpose (3D reconstruction, compressibility-assessment, Doppler, etc.). Alternatively, several information can be collected in one scan. It may be necessary in some instance to process, manually or with the help of algorithms, to differentiate between the different sequences obtained in one scan.

Applying compression while imaging is not only advantageous post-treatment to assess vein compressibility. Preferably imaging while applying some pressure onto the tissue is performed before treatment. Preferably, the compression force is chosen in order to collapse the vein (either completely or partially, preferably to have the target substantially in the same state as during treatment). Most preferably, the minimum compression force needed to completely collapse the vein or to collapse it as in treatment is used. Preferably, compression is directly applied by pressing with the imaging probe (in particular a ultrasound hand-probe) onto the patient or by performing the scan with a treatment head, preferably substantially identical to the treatment head which will be used for a subsequent treatment. Compression can be applied either in specific points or continuously during a scan of the vein.

Typically, applying compression is advantageous because depth under skin of the target may be an important parameter for certain ultrasound treatments. For example, if the target is close to the skin, the used power may have to be limited to avoid skin burns. Moreover, different types of ultrasound pulses can be used to treat at different depths (usually, higher ultrasonic frequencies are used for more superficial targets). In clinical practice, physicians measure the depth under skin without applying compression. This can lead to inconsistencies between the depth measured before treatment and the actual depth during treatment, especially if compression is applied during treatment, for example to collapse a vein.

Several options are available to evaluate the depth under.skin with compression, which preferably substantially correspond to the depth under skin during treatment.

For example, if only the target depth with compression is of interest, the physician may measure the target depth with compression. Alternatively, in order not to change the clinical practice, the depth without compression and the depth with compression can be measured (either at specific points or during whole scans) on several patients and a model can be trained to estimate depth under the skin with compression based on the measurement without compression. Models used may comprise a linear regression and/or a neural network taking into account the depth measured without compression and a B-mode picture and having the depth under skin with compression as target function.

Then, treatment planning and/or patient recruitment is preferably based on the depth with compression (either measured or estimated). Moreover, if several treatment heads or therapy transducers can be used with the device, a choice of a therapy transducer may be made based on the depth with compression.

Preferably, the depth with compression is also a feature used in the treatment planning and/or automatization algorithms.

As mentioned above, the difference or the ratio between the depth under the skin with and without compression may be used as a parameter in treatment planning and/or evaluation. Indeed, it may give indirect information on tissue composition.

Preferably, the visualization of the acquired data is be performed on the medical device which served for the acquisition, and/or on another medical device (e.g. a medical device which serves for a therapy), and/or on a computer or mobile support, preferably using an intranet or internet connection to a remote of local database when the visualization tool is not on the medical device which served for the acquisition. Data can also be transferred using removable storage means (USB sticks, external hard drives, and other means known in the art).

Preferably, the visualization tool includes an interface to review the acquired sequence (with respect to time) or the acquired data (with respect to space), or a reconstructed model, e.g. a reconstructed volume, (with respect to space).

Preferably, images or videos are extracted from the acquisitions and linked to the graphical elements displayed when visualizing the 3D model of the structures of interest (e.g. markers 72, 73, see Fig. 9).

In the example of Fig. 9, when a user clicks on a marker 72, 73, a short video sequence may be displayed showing the sequence during which the user tries to compress the vein. These images or videos can either automatically be extracted from the acquired sequences, or manually selected by the user from the acquired sequences.

Fig. 10 shows a further example of an interface to manually extract information from acquired data. The interface comprises a control 87 which can be used to load acquired data. A slider 83 enables navigation in time within the acquired data. The corresponding position 82 is represented along the vein V. It is possible to select a time interval 84, represented by an A-B segment on the slider 83. Furthermore, controls 88', 88", 88''' can be toggled to indicate the compressibility or flow information at the corresponding position or position. In this example, a dedicated button 85 enables to set the A-B time interval to the maximum interval around a current time during which the position along the vein stays constant. This is advantageous when extracting a video sequence corresponding to a compressibility assessment, for example, and to link it to a compressibility assessment. Alternatively, a second control 86 allows to define A-B as the current position (A and B both moved to the current position of the slider, and the extracted sequence is thus a screenshot). An image 89 representing the vein V at the time determined by the slider 83 and at the position 82 is also shown on the interface.

Preferably, the interface enables to review the vein at different timepoints. Preferably, it is possible to overlay the information at the different timepoints. Preferably a user interface enables the visualization of the evolution of the structure vein at different timepoints.

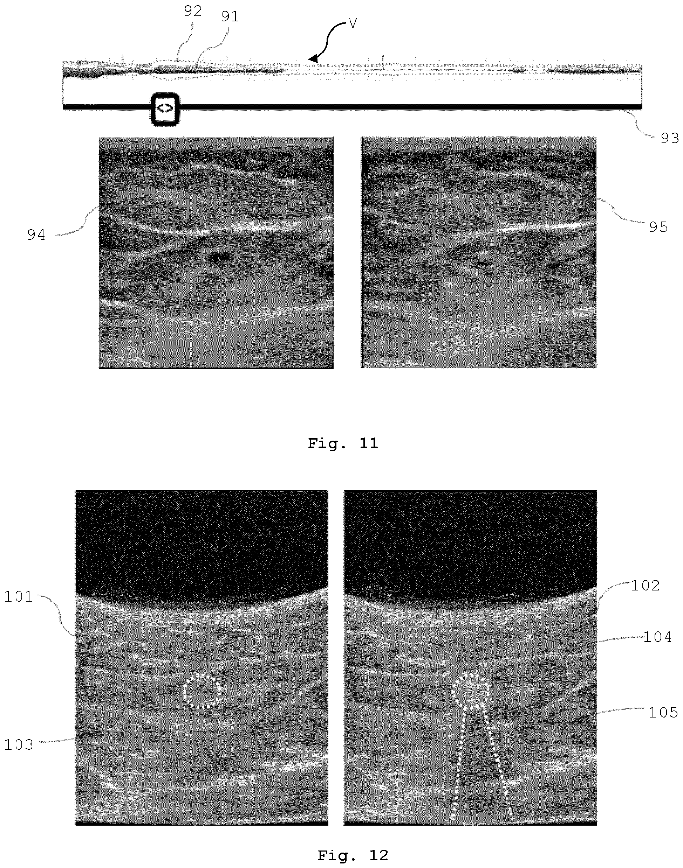

Figure 11 shows an example of interface enabling the visualization of slices extracted from the reconstructed volumes before and after a treatment. A first image 94 shows a body structure before a treatment and a second image 95 shows the body structure after a treatment. The vein V is represented as a volume 91, which corresponds to the vein V after a treatment. A profile of the vein V corresponding to a state before treatment is represented as a dotted line 92. Navigation along the vein may be done using the slider 93.

If the structure of interest comprises a tumor (e.g. a thyroid nodule), the volume of the tumor is preferably measured and plotted with respect to time. Such a time-dependent assessment can indicate whether a tumor grows and at which pace in the context of pathology follow-up. Furthermore, in the context of treatment outcome assessment, it may also enable to quantify the shrinkage of the tumor. Reconstructed volumes comprising elastography and/or duplex data may help identifying portions of a tumor or other tissue that were insufficiently treated.

Fig. 12 shows an example of hyperechoic mark (HEM) occurring in a treatment and shows a first B-mode image 101 captured before an ultrasound pulse (not shown) and a second B-mode image 102 captured after delivery of an ultrasound pulse (not shown). A vein 103 is visible on the first image 101, i.e. before a pulse is delivered. After delivery of a pulse at the location of the vein V in a treatment slice corresponding to the image plane of the first image 101 (i.e. the second image 102 is taken a position which is substantially the same as the first image 101), the zone contains a hyperechoic mark 104, which also induces a shadow 105 on the B-mode image, since it prevents the imaging ultrasound beam from reaching the tissue below.

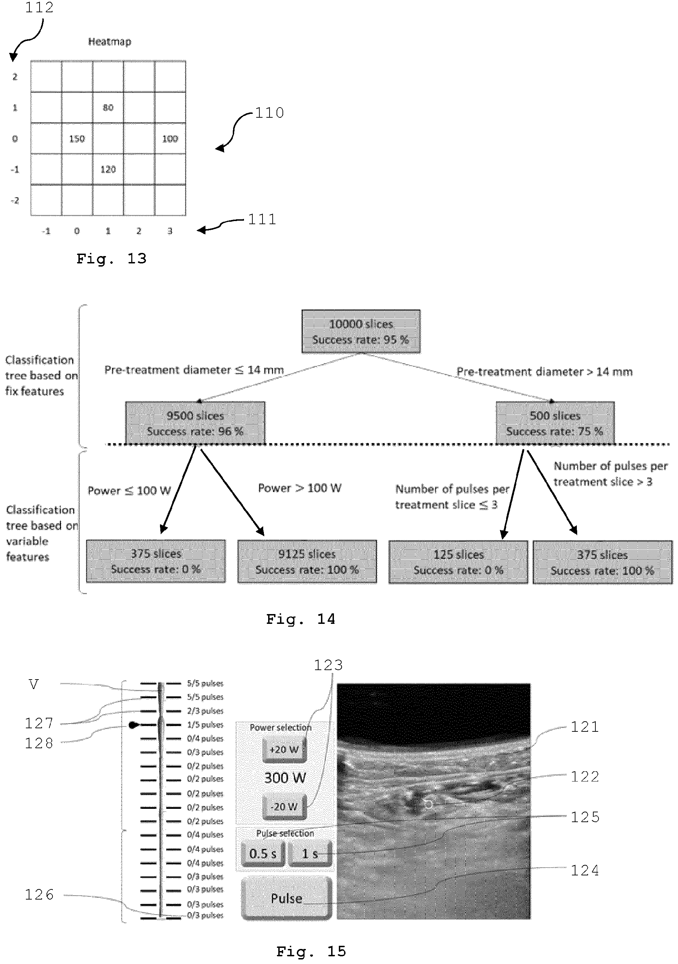

Fig. 13 shows an example of a heat map 110. The heatmap comprises a first axis 111 and a second axis 112 which indicate, on a relative scale, a position in the tissue. For any coordinates defined by the first and second axis 111, 112, a delivered power may be recorded. Here, illustratively, a power of 80 W was delivered at position (1,1), 150 W at (0,0), 120 W at (1,-1), and 100 W at (3,0).

It will be understood that it is also possible to record the same data in the form of a table, as is well known in the art. It is also possible to record average information of one or several parameters, in particular of all parameters. For example, for the heatmap shown in Fig. 7, an average horizontal position is 1 and an average vertical position is 0. The average power delivered is 90. Further statistical parameters known in the art, e.g. median, minimum value, maximum value, standard deviation, etc., may be employed.

Fig. 14 shows an example of classification tree in which the classification is done based on fixed features in a first stage and based on variable features in a second stage. In shown example, the patient may first be classified based on the mean pre-treatment diameter of the targeted vein.

Then, for a vein having a pretreatment diameter greater than 14 mm, it would be recommended to the user to deliver more than 3 pulses per slice. For small veins (with a pretreatment diameter of less than 14mm), it would be recommended to treat with a power greater than 100 W.

As an example of AI model, a two-floors classification tree (as shown in Fig. 14) may be employed. For example, with simple machine learning algorithms such as classification trees, the distinction between fixed and variable features may be implemented by training a classification tree to predict success or failure at the scale of the slice based on only fixed features (e.g. age, BMI and/or pre-treatment diameter of the vein). Each leaf of the tree may serve as the root of another classification tree based on variable features (such as number of pulses and/or mean power).

This may be advantageous because classification trees enable to classify patients and allow recommendation of treatment features for each individual patient based on the classification.

As further example of AI model, a 3-floors classification tree may be provided and serve to plan a HITU treatment.

For example, a 3-floors classification tree may be trained to predict an outcome at certain slice based on fixed features of a patient (e.g. age and/or BMI). Each branch of the tree could serve as the root of another classification tree based on the fixed features of a slice (e.g. diameter and/or depth under skin), each of which may serve as the root of another classification based on variable features (e.g. number of pulses and/or mean power). Based on such a 3-floors classification tree, a software could be designed to plan a HITU treatment for each treatment slice. For example, a first portion of the tree is selected which corresponds to the fixed features of the current patient. Then, for each treatment slice, a sub-portion of this first portion is selected which corresponds to the fixed feature of the current slice. Finally, the values of the variable features are chosen so as to make the current treatment slice fall in the leaf leading to the highest average vein shrinkage for example. This treatment planning algorithm is given as a suitable and explainable example, but other algorithms may be used (e.g. a combination of a grid search algorithm on the treatment parameters with a regressor predicting the value of the outcome metric for given treatment parameters).

Fig. 15 shows a further example of treatment interface. A reticle 122 (i.e. an overlay which shows the position of the focus in the image) is overlaid on a live B-mode image 121 to assist targeting. A user may choose the type of pulse with a first control 125 and increase or decrease the power using a second control 123. Optionally, one or both of controls 123, 125 may light up when, based on a simulation/algorithm, a change in power is recommended (for example, depending on the characteristics of the HEM, such as area, height, brightness). Here, however, the power is automatically adapted in such a case. The use is informed, via the interface, when the power is been changed. Via control 123, the user may still manually adapt the power. Pulses may be triggered with a third control 124. The vein V is displayed on the user interface in the form of a reconstructed volume as described herein. The interface further displays a recommendation 126 of a number of treatment pulses for each treatment slice as well as the actually delivered pulses at any given time. In some embodiments, a recommendation may be made in terms of number of pulses that generate an HEM, meaning that only pulses that generate an HEM are counted towards the recommendation.

Additional information may be displayed, for example a location of tributaries, junctions, and/or sensitive structures. A recommended type of pulse and/or a recommended number of pulses may be displayed, as is a the number of delivered pulses, for each treatment slice which is shown by markers 127. The current location of the treatment head along the vein is also displayed by a second marker 128. Here, only the position of the treatment head along the vein is of interest.

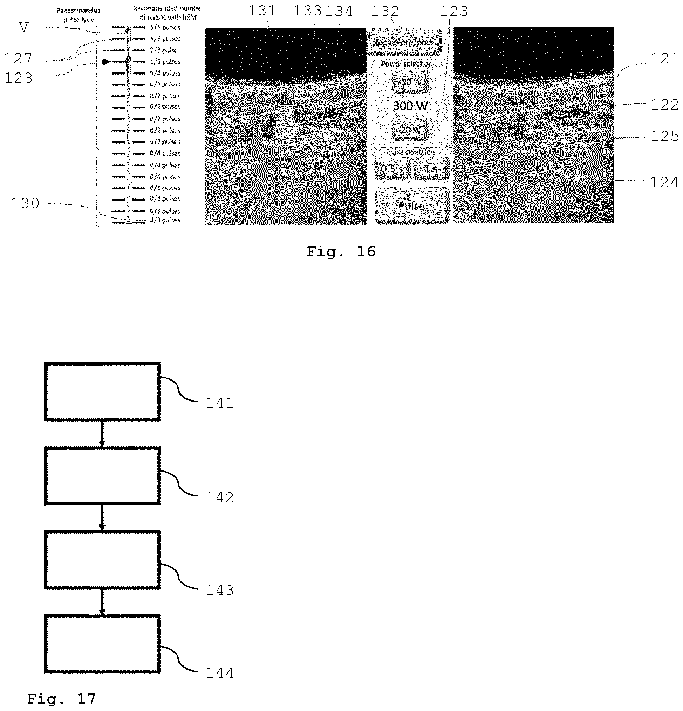

Fig. 16 shows an further embodiment of an interface which is based on the interface shown in Fig. 15. Here, the treatment interface provides a user with local treatment recommendations (i.e. for each treatment slice), automatic power suggestion and a HEM review interface. The interface comprises a first image display 121 and a second image display 131. Here, the first display 121 shows a live B-mode image. A reticle 122 is overlaid on the live B-mode image shown on the first display 121 to assist targeting. A pulse control 125 lets a user choose a type of pulse, here between pulses with a duration of 0.5 s or 1.0 s. A further control 123 allows an increase or decrease of the power.

As described above, HEM,characteristics (such as area, height, and/or brightness) may be analyzed by an image analysis software, for example a segmentation algorithm such as a U-net neural network, in order to recommend to the user an increase or decrease in power, which may be indicated e.g. by a change (e.g. in color or brightness) of the controls 123. Additionally or alternatively, the power may be automatically adapted and the user may be informed that the power has been changed. Pulses can be triggered with a pulse control 124.

In a similar fashion as shown in Fig. 15, a vein V is shown as a reconstructed volume. Additional information such as location of tributaries, junctions, and/or sensitive structures may be displayed as well. A recommended type of pulse, e.g. 1 s or 0.5 s pulses, may be displayed either on a dedicated display (not shown) or by color/brightness of controls 125. Furthermore, the number of delivered pulses and the recommended number of pulses 130 in each vein cross section 127 is shown. A current location of the treatment head along the vein is also displayed by a marker 128. Here, the second display 131 shows a post-pulse image captured after a previous pulse. A HEM 133 is visible and was automatically segmented. The resulting outline 134 is overlaid on the post-pulse image shown on the second display 131. A button 132 enables to switch between the post-pulse image and a corresponding pre-pulse image shown on the second display 131.

Algorithms may be used to recommend treatment of a part of the vein with a different therapy than another. For example, an al-gorithm may determine that HITU is preferable for a first portion of a vein while a second portion of the vein may be treated with sclerotherapy.

Fig. 17 shows an exemplary flowchart of a patient management process. A pre-treatment acquisition is performed 141. Optionally, the patient may subsequently be placed in treatment position. A treatment registration acquisition is performed 142. Treatment registration acquisition is particularly advantageous where the pre-treatment acquisition is not performed with the patient in the same position as for the treatment. Treatment registration acquisition 142 may be omitted in cases where the pre-treatment acquisition is performed just before treatment and in the same position. A treatment is then performed 143. Finally, a post-treatment acquisition 144 is conducted. The post-treatment acquisition 144 is preferably conducted in the same position as the pre-treatment acquisition 141 and may allow to follow the outcome of the treatment.

It will be understood that all steps 141-144 may be repeated if desired or required. In particular, step 144 is advantageously repeated to evaluate outcome of the treatment at different timepoints.

Treatment registration acquisition 142 and Treatment 143 are preferably performed with the patient in the same position.

Preferably, at least one landmark is identified before the treatment registration acquisition 142. For example, after the pre-treatment acquisition 141 or before the treatment registration acquisition 142. This may be advantageous because it enables the user to consider them during the treatment registration acquisition 142.

Preferably, a software is used to review the pre-treatment acquisition 141, in particular in the form of a acquired data or reconstructed model, and may also allow to annotate landmarks and/or to review annotated landmarks.

The pre-treatment acquisition 141 is preferably performed with an ultrasound hand-probe, in particular where a treatment 143 is performed with HITU.

The hand-probe and the treatment head may be tracked with the same system during treatment (e.g. an EM tracking system or an optical tracking system), which may allow for more accurate comparison between positions of imaging and treatment data. This may also enable the user to find the landmarks with the hand-probe which may provide better image quality and easier handling compared to the treatment head. Alternatively, the landmarks may also be found with the treatment head.

Preferably, the treatment head comprises at least one imaging modality which may be used to acquire the position of landmarks during the pre-treatment acquisition 141. Additionally or alternatively, the treatment head may comprise a different imaging modality which enables to see the same landmarks.

It is also possible to use external fiducials, alone or in combination with anatomical landmarks, for example electromagnetic sensors and/or markers for optical tracking placed on the patient. By way of example, a sensor and/or markers for optical tracking may be placed on a knee pad or maintained on the leg by a bandage or any suitable arrangement. Preferably, at least an external fiducial is substantially fixed with respect to the anatomical structure on which the treatment is performed. For example, if the treatment is a HITU treatment of a vein in the thigh, at least an external fiducial is preferably fixed on the thigh, between the groin and the upper part of the knee, since the presence of the femur prevents important deformation of the leg segment along its proximal-distal axis. Similarly, in the case of a HITU treatment of a vein located in the calf, at least an external fiducial is fixed on the calf, between the lower part of the knee and the ankle.

A structure of interest may be deformed between pre-treatement acquisition 141 and treatment 143. For example, a GSV may be treated from a patient's groin to an ankle, wherein the patient bends the leg while in a treatment position. Thus, it is possible to, as part of any method according to the invention, subdivide the structure of interest into substructure for the purpose of analysis and evaluation. It will be understood that in particular reconstruction and registration of imaging data may be performed separately for substructures. Subdivision into substructures is preferably done into substructures which are not deformed, e.g. between two positions. For example, a GSV may be subdivided into a first part above and a second part below the knee.

Deformations may also occur during a treatment due to injection. In particular in such cases, the tracking system may be used to estimate the position of a focus along the vein. A position in the plane orthogonal to a longitudinal axis of a vein may be estimated based on a vein detection algorithm. A vein detection algorithm may segment the vein on a current image and/or use a spatial sequence of images to detect the vein, using sequential neural networks for example, and/or perform a registration based on images acquired before injection at the current location along the vein.

Indicating the position of the landmarks when the patient is in treatment position may be done during imaging on a live image with a control, for example a footswitch, buttons on a touchscreen, and/or buttons on the hand-probe. Additionally or alternatively, landmarks may be positioned and/or displayed after acquisition. For example, a manual registration interface may be used (see above, Fig. 8) to this end.

Additionally or alternatively, landmarks may be identified on pre-treatment acquisition data. An automatic image-processing algorithm may be used to find said landmarks in the treatment registration acquisition. Preferably, even when at least one position of a landmark is indicated on a live image as described above, it is possible to review and correct the registration manually or automatically after acquisition. For example, for registration of imaging data of a vein, the junctions with tributaries is an advantageous set of anatomical landmarks.

If the patient moves or is changed in position (e.g. by movement of a treatment table), the registration of the treatment head's coordination system with the pre-treatment acquisition data is preferably repeated. For example, a patient may be placed in a Trendelenbourg position during a treatment.

A current position of the treatment head within a reconstructed volume may also provide further advantages. Data sufficient to train the recommendation algorithms described herein is preferably performed in all methods according to the invention and may include the position of the treatment head with respect to the structure of interest. Additionally or alternatively, at least one image captured at a location may be displayed, either during a pre-treatment scan or during the treatment registration acquisition, based on a current location of the treatment head.

For example, in the context of a HITU treatment, the pre-treatment acquisition and/or the registration acquisition are preferably performed with a hand-probe, while the visualization during treatment is enabled by an ultrasound probe embedded in the treatment head. In this case, the ability to locate-the vein is usually easier on the pre-treatment acquisition and/or on the registration acquisition because the vein has not yet been collapsed by the effect of the beginning of the treatment, and the hand-probe is in direct contact with the skin, which may not be the case with the treatment head. Moreover, an infiltration may have been performed before the treatment. Thus, it is possible to display at least an image taken from or reconstructed from the pre-treatment and/or registration acquisition at substantially the current position of the treatment head along the vein to help the user locating the vein on the current image with the treatment head. Typically, this is advantageous because the user can see the relative location of the vein with respect to other structures (e.g. fascia) on the pre-treatment and/or registration acquisition. These structures may be more easily visible than the vein on the current image with the treatment head, thus serving as visual landmarks for estimating the vein position. This process of finding the vein on a current image with a treatment head based on at least a previous image captured at substantially the same location along the vein during a pre-treatment and/or registration acquisition may be automated using image registration algorithms as known by the person skilled in the art, thus providing the user with an instantaneous estimation of the vein location.

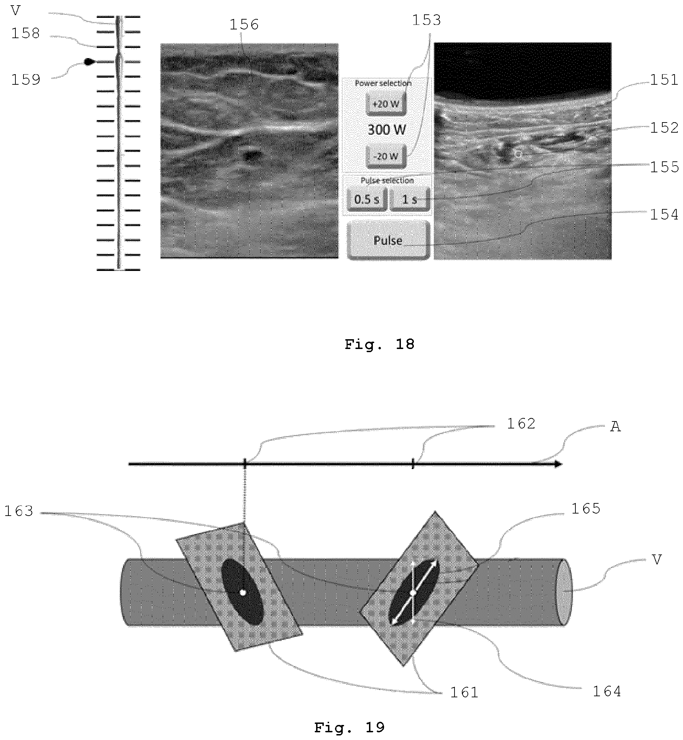

Fig. 18 shows a further example of a treatment interface. The shown interface is substantially similar to the interface shown in Fig. 16 and comprises a first display 151 and a second display 156. The first display 151 shows a live B-mode image.'A reticle 152 is overlaid on the live B-mode image on the first display 151 to enable targeting. The user can choose the type of pulse with a pulse control 155 and increase of decrease the power using a power control 153. Power adjustment recommendations, as described in the context of Fig. 16, are possible with the shown interface. Pulses can be triggered with a further control 154. The vein V is shown as a reconstructed model. The current location of the treatment head along the vein is also displayed 159.. The currently treatment slice, where the treatment head is located, is marked by marker 159. An image of the selected treatment slice which was acquired during a pre-treatment acquisition and/or during the treatment at substantially the same location, is automatically shown on the second display 156. Preferably, the image is part of an acquired sequence or an extracted slice. The image, which maybe high-quality image, for example acquired with a hand-probe instead of the treatment head, which usually results in a better image quality since the hand-probe is in contact with the skin, may also be displayed continuously or continuously accessible.

Fig. 19 schematically shows the acquisition of B-mode images 161 of a vessel V. Here, two different positions 162 along the longitudinal axis A of the vessel are assigned to the images 161 based on the position of the center 163 of the vein V. The actual diameter 164 of the vein V is estimated for a position 6 based on the diameter 165 on the image 2.

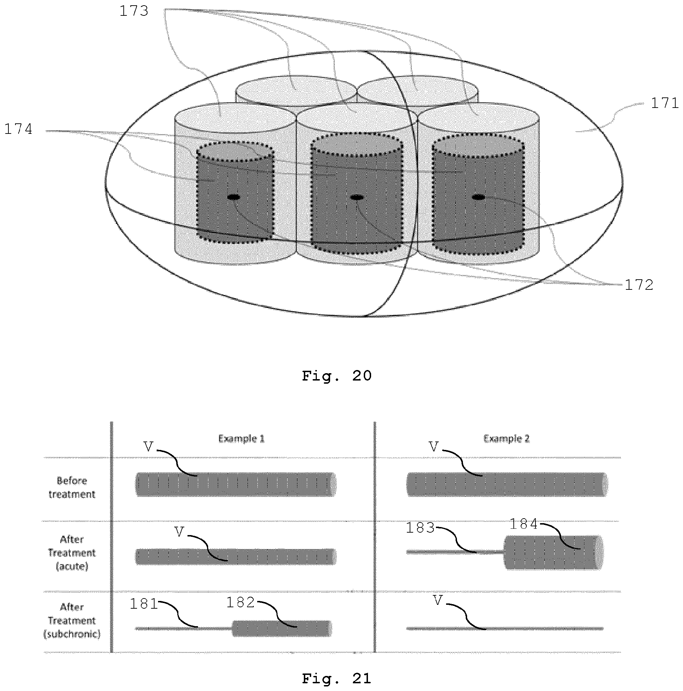

Fig. 20 schematically shows the treatment of a tumor. In the context of tumors (for example malignant tumors), dedicated imaging modalities can be used to assess the local efficacy of a therapy. A ultrasound contrast agent may be injected after a HITU treatment of a tumor 171. As a result, a three-dimensional induced by the treatment may be assessed as a hypoechoic region in B-mode imaging. If a lesion was expected having a lesion volume 173 around a set of positions 2 and the actual lesion volumes 4 are measured, an outcome metric may be determined by calculating the ratio between the measured and the expected volume of the lesion induced by HITU. Other analysis techniques, for example circulation of biomarkers, may be used to assess the efficacy of a therapy on a tumor.

Fig. 21 shows schematically a first and a second example of a vein which was treated and wherein an outcome metric is computed based on several acquisitions at different timepoints before and/or aftertreatment, aggregated by a function. In both examples, substantially identical veins before treatment are shown.

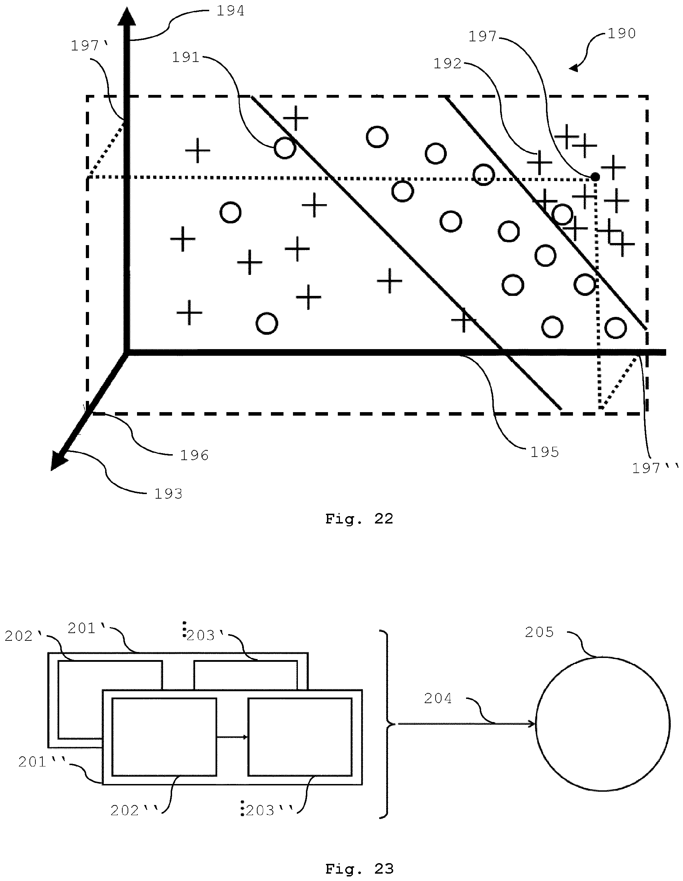

In the example 1, the vein V is only slightly shrunk in acute and as time advances, a first portion 181 becomes sealed close, while a second segment 182 one stays open. In this situation, the most advanced timepoint may be considered more relevant. In the example 2, a first segment 183 of the vein V is completely shrunken in acute while the second segment 184 is even inflated in acute. At a subchronic timepoint, the vein V is completely shrunken and sealed closed along all its length. In this situation, given the evolution of the vein, it may not be possible to know if the vein has shrunken because of a proper treatment in the region which is shrunken, or because the segment on the right was successfully treated. In this situation, if the outcome metric is computed to ultimately train a predictor of the shrinkage based on local treatment parameters, it may be more advantageous to consider all the information and to discard the second segment 184 from the analysis for example. Fig. 22 shows a graph 190 depicting treatment characteristics of treatment slices which are part of a treatment. Circles 191 depict treatment slices of HITU treatments of veins assigned to a "success" class, and the crosses 192 depict treatment slices assigned to a "failure" class based on the value computed for an outcome metric, here the local vein shrinkage in each treatment slice. A supervised metric learning was used. The treatment characteristics are plotted with a first axis 193 representing a combination of fixed features (e.g. sex, age, height, weight, the patient having a profession requiring standing position, comorbidities, current treatments such as anticoagulants, reflux type such as systolic or diastolic, pre-treatment vein diameter in standing position, history of other treatments performed on the target, location of fibrotic tissue in the vicinity of the target which may result from a previous treatment for example)), and a second axis 194 and a third axis 195 representing one variable feature each (e.g. number of pulses delivered in the treatment slice and mean power of the pulses delivered in the treatment slice).

To optimize a treatment, a subspace 196 is defined in terms of the fixed feature, i.e. a plane orthogonal to the axis 193 for each treatment slice (only one such plane being shown for clarity). In the subspace 196, a vector 197 with vector components 197', 197" corresponding to the two variable features are chosen. The vector 197 is chosen of values which are achievable by a medical device used to perform the treatment, and falls within a cluster containing mainly treatment slices 191 which are assigned to the "success" class.

Fig. 23 schematically shows a method for training a model, in particular by performing supervised training. Two datasets 201', 201'', each comprising an input feature vector 202', 202" representing a -number of HITU pulses delivered in each of a series of treatment slices along a vein. Additionally or alternatively, patient characteristics such as the age and BMI of the patient may be represented by the input feature vectors 202',202". Furthermore, each dataset 201', 201" a target feature vector 203', 203'' depicting the outcome of a treatment delivered according to the input feature vector 202',202". The target feature vectors 203', 203" are computed based on an outcome metric. For example, the target feature vectors 203', 203" may be a collection of shrinkage values in each of a series of treatment slices along a vein or the mean shrinkage among a series of .treatment slices along a vein. Thus, each dataset 201',201" relates treatment characteristics to the corresponding treatment outcome. Based on a plurality of datasets 201', 201'' (preferably at least 100, most preferably at least 500), a model training 204 is performed to obtain a trained model 205 able to predict a target feature value (or a range of target feature values, preferably with associated probability distribution) based on an input feature vector 202', 202". A part of the data may be used for model test and/or validation and not used for training.

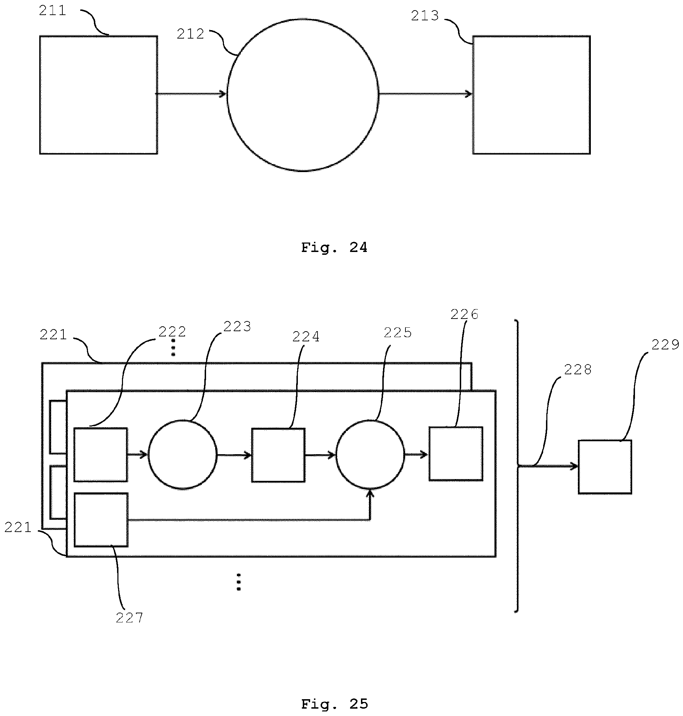

Fig. 24 schematically shows the use of a trained model 212. For example, the model 212 may have been trained as shown in Fig. 23. The model 211 allows to predict a target feature value 213 based on an input feature vector 211.

Fig. 25 schematically shows a selection process of a treatment characteristic using a grid search algorithm. Combinations 221, corresponding to treatment scenarios, are tested by predicting the target feature value 224 (e.g. the shrinkage of a vein) of a treatment based on an input feature vector 222 and a trained model 223. Additional computations 225 are preferably performed based on additional feature values 227 (e.g. by subtracting the treatment duration multiplied by a weighting factor), resulting in a value 226 of an objective function. Then, a selection process 228 (e.g. choosing the combination maximizing the objective function) returns one value 229 of at least one treatment characteristic. In particular, a depth under skin of the target (e.g. a vein) may be part of the input feature vector, for example as a fixed feature, but may also be an additional feature value 227.

The values of the fixed features in the input feature vector 222 and/or in the additional feature values 227 are substantially the same in all combinations 221 but can also vary (for example, to test the sensibility of the choice of the optimal combination to the values of the fixed features). If the value of the objective function 226 varies disproportionately when changing the power of a HITU pulse by one milliwatt, it may mean that the optimum found is artefactual and should not be chosen. In such an example, another value for the power may be chosen, which does not necessarily result in the global optimum of the objective function but in a local optimum.

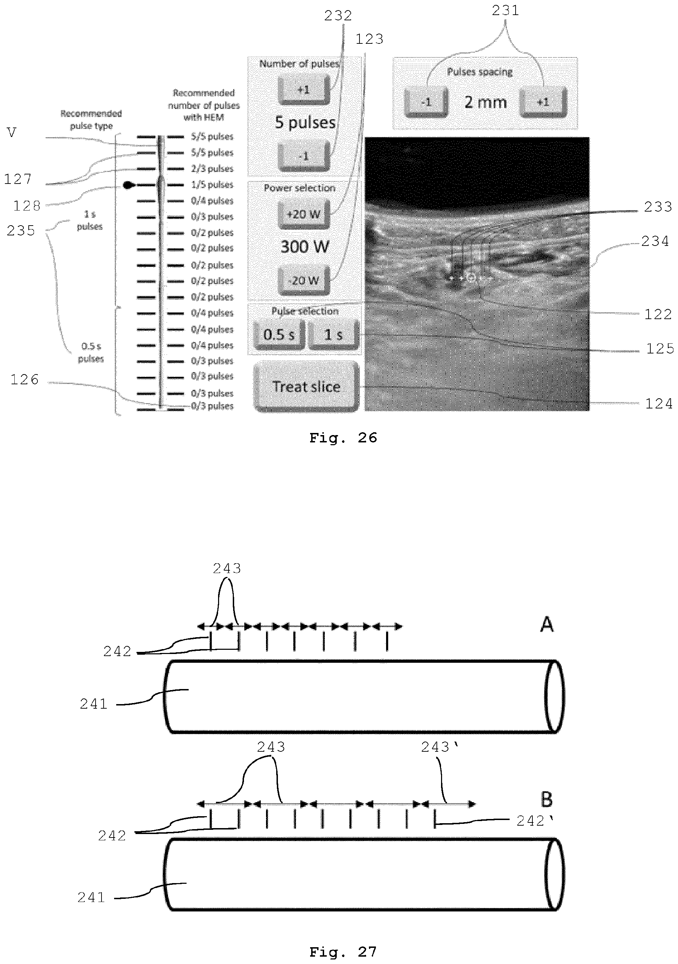

Fig. 26 shows a treatment interface similar to the one shown in Fig. 15. Identical features using the same reference sign are not described again for clarity. The interface comprises, the control 124 triggers the definition of a certain number of treatment sites 233, here located laterally in the plane corresponding to the image and centered on the current location 234. Subsequently, one HITU pulse per site is delivered, which may be done automatically or manually. Subsequently, the treatment head is preferably moved to the lateral location at which the control 124 was activated. This is advantageous because, if the vein is orthogonal to the current imaging plane, it enables the user to keep the vein in the center of the screen when moving from one treatment slice to another. Thus, the user can successively activate the control 124, move to the next slice, and repeat without having to adjust the lateral position. The lateral spacing between treatment sites may preferably be modified by the user with dedicated controls 231. The default value is 2 mm but may be between 1 and 5 mm for example. The number of treatment sites defined when clicking on the control 124 corresponds here to the number of pulses or the number of pulses with HEM 10 advised by a treatment planning algorithm. Alternatively, this number may be defined by the user in which case a default number of pulses may be set and may be 1 or 3 or the diameter of the vein divided by the spacing or another value (e.g. 2 mm). In general, the number of pulses per treatment slice is between 1 and 10 and ideally lower than 6. The user may adjust the number of pulses per slice using a dedicated control 232. In some embodiments, the interface further comprises a dedicated control not shown here enabling to repeat a pulse at the location of the previous pulse delivered. This is advantageous if the previous pulse did not lead to the expected tissular response (e.g. an HEM). If an automatic algorithm is able to detect the expected tissular response (e.g. an automatic HEM segmentation algorithm, or a classifier), it may automatically modify the sequence or suggest appropriate modifications to the user. For example, it may adapt or suggest to adapt the power (e.g. increase the power if no HEM is observed after a pulse, or decrease the power if a HEM bigger than a certain area, or higher than a certain position, is observed after a pulse) and/or modify or suggest to modify the number and/or position of pulses (e.g. stop the sequence if a HEM bigger than a certain area, or higher than a certain position, is observed after a pulse, or repeat a pulse at the current position if no HEM is observed after a pulse, preferably with a higher power). The interface further comprises an indication of a recommended pulse length 235 in relation to the location on the vessel V. The user may select the pulse type using control 125 independently of the recommendation 235. The pulse power may also be set using control 123. An additional control (not shown) may also exist to control a number of treatment slices which are automatically consecutively treated. Another control (not shown) may also exist to change between different orders of treatment of the treatment sites (e.g. from left to right, or from right to left, or by alternating between the leftmost and rightmost treatment sites untreated yet).

In the Figs. 27-29, for clarity, reference signs may not be shown multiple times when referring to the identical elements, but it will be understood that certain elements may be shown more than once in certain figures.

Fig. 27 shows an example of locations of the segments 243 with respect to the treatment slices 242 along a vein 241. In panel A, all segments 243 are centered on treatment slices 242. The lengths of the segments 243 may adapt to the local spacing between treatment slices. In panel B, the segments 243 are centered on pairs of the slices 242. Since there is an odd number of treatment slices 242, a last segment 243' is not centered on the treatment slice 242' it encompasses. An outcome metric may or may not be computed in untreated sections of the vein.

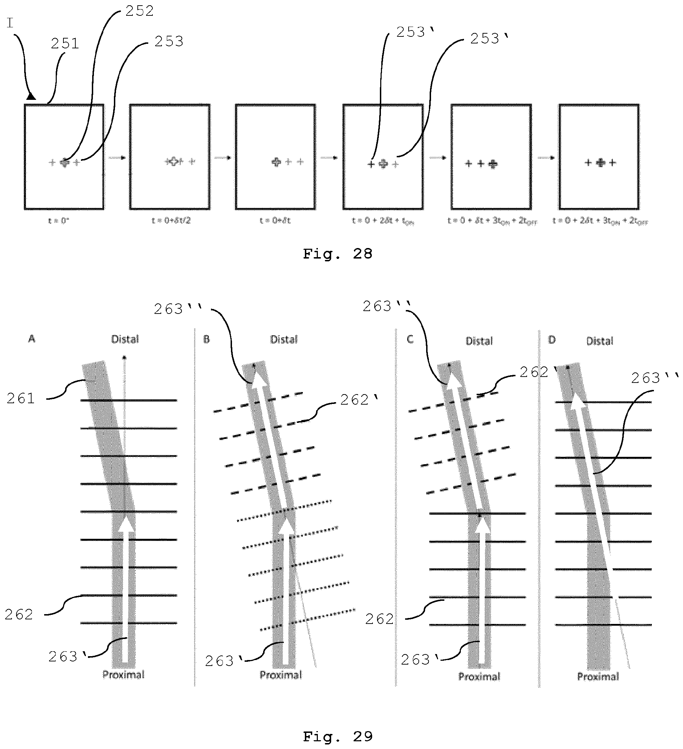

Fig. 28 shows an example of an evolution of portion of a HIFU treatment interface I comprising a view 251 of an anatomical scene comprising a target. Furthermore an overlay 252 indicating a characteristic point or zone of the acoustic field (e.g. the acoustic focus) is overlayed, preferably at a fixed position, onto a live image. Representations 253 of locations of the treatment sites are shown and may be depicted, e.g. in a different color or greyscale, depending on whether they represent treated or untreated sites.

The locations of the treatment locations are preferably fixed with respect to the anatomy, thus, they move on the image as the treatment moves. t represents the time since the user activated the control triggering the treatment of the sequence of treatment sites. At t = 0+, i.e. just after the user activated the control, the locations of the treatment sites are defined and preferably displayed. In this example, there are three treatment sites centered on the current location. $\delta t$ represents the time necessary for the treatment head to move from one site to another. tON represents the duration of a pulse and tOFF the duration of the pause between consecutive pulses. In this example, tOFF is greater than $\delta t$, thus the displacements are included in the pauses.

In the shown example, the treatment sites are equally spaced, the pulse and pause duration are the same for all pulses, and a single pulse may be sufficient to treat each treatment site. At t= 0+$\delta t/2$, the treatment head is halfway between the starting position and the position of the first treatment site. At t = 0+$\delta t$, the treatment head is at the position of the first treatment site. At t = 0+2$\delta t$+tON, one pulse has been delivered on the first treatment site, thus a cross 253' representing the location of the first treatment site is displayed in bold, while an untreated site 253'' is represented as a thinner cross. The treatment may be moved to the location of the second treatment site. At t=0+$\delta t$+3tON+2tOFF, one pulse was delivered on each of the tree sites. At t=0+2$\delta t$+3tON+2tOFF the treatment head moved back to its initial position.

Fig. 29 shows an illustration of a modification of treatment progression direction during a HIFU treatment of a vessel 261. First, the treatment head is positioned on the proximal part of the vessel and the treatment progression direction 263' is defined based on the local orientation of the vessel, thus defining treatment slices 262 as shown in panel A.

Then, during the treatment and as illustrated in panel B, the treatment progression direction 263' is modified to account for a change in the direction of the vessel. This may result in a recomputation of the treatment slices 262, wherein the treatment progression axis is redefined in the proximal and in the distal parts of the vessel 261, thus resulting in new treatment slices 264 arranged with respect to treatment progression direction 263". This results in a particularly easy-to-implement computation.

Alternatively, as illustrated in panel C, the location of the treatment slices 262 is kept constant in a first portion of the vessel, here in a proximal part, and recomputed in a second portion of the vessel, here in a distal part. Here, moving from the proximal set of the slices 262 to the distal set of the slices 262' (or the opposite) may result in a translation and/or a rotation of the treatment progression direction 263" with respect to treatment progression direction 263' in order to account for the change in angle of the treatment slices 262, 262'. This method is advantageous because it may enable to come back to previously treated treatment slices 262 with the original orientation.

Panel D shows an alternative variant, similar to panel A, in which the location of the treatment slices is not recomputed but the treatment progression direction 263" is modified.

Further disclosed is a computer-implemented method of training a machine-learning model for determining an outcome metric associated with a procedure, in particular a High-Intensity Therapeutic Ultrasound (HITU) therapy, or another type of therapy including radiofrequency ablation, laser ablation, surgery or sclerotherapy, or a subpart of a therapy, in particular a HITU pulse or a series of HITU pulses or a local anesthesia, or the positioning of a medical device, or a change in position of a person, or daily activities following a therapy (follow-up period), or a combination thereof. The method comprises obtaining pre-procedure digital data associated with an anatomical structure of a patient before the procedure, the pre-procedure digital data comprising, or being derived from, data obtained in a medical imaging procedure. Post-procedure digital data associated with the anatomical structure after the procedure is obtained. Based on, at least in part, the pre-treatment digital data and the post-treatment digital data to predict a local or spatially dependent outcome metric reflecting a physical change, preferably a physical effect of the procedure, the machine-learning model is trained.

In one embodiment of the method, the digital data comprises at least one of a digital image, in particular a B-mode image; acquired data comprising a set of digital images, in particular B-mode images; a reconstructed volume comprising a voxelized volume generated based on acquired data, the voxelized volume comprising data derived from the acquired data, in particular using interpolation; and/or a model, preferably a three-dimensional model, of the anatomical structure, in particular comprising a mesh, point cloud and/or label map.

In some embodiments of the method, the anatomical structure comprises at least one of an internal-body structure; an organ; an essentially tubular anatomical structure, in particular an essentially hollow tubular anatomical structure, such as a blood vessel, in particular a vein; a pathological anatomical structure, such as a varicose vein, a tumor or a thyroid nodule.

In some embodiments, the pre-procedure and post-procedure digital data comprise position and/or orientation data suitable to at least partially spatially register the pre-procedure and post-procedure digital data before computing the outcome metric along at least one axis.

In some embodiments, the post-procedure digital data comprises or is derived from data obtained in a repetition of at least part of the pre-procedure data acquisition process.

In some embodiments, the outcome metric indicates at least one of a difference between the diameter of the anatomical structure before and after the treatment, a shrinkage of the anatomical structure, an incompressibility of the anatomical structure, an absence of reflux in the anatomical structure, and/or an absence of flow in the anatomical structure.

In some embodiments, the outcome metric is computed at at least two timepoints based on pairs of a pre-treatment acquisition and at least two post-procedure acquisitions, wherein the timepoints are preferably one of acute, sub-chronic and/or chronic timepoints.

In some embodiments, the training of the machine-learning model is at least partially based on at least one fixed feature or patient parameter. The fixed feature may be selected from sex, age, height, weight, body mass index, profession requiring standing position, comorbidities, current treatments, reflux type, in particular systolic or diastolic reflux, pre-treatment vein diameter in standing position, depth-under skin of the anatomical structure during the pre-procedure data acquisition process, depth-under skin of the anatomical structure when the patient is in position for the procedure, pre-treatment volume of a tumor, history of other treatments performed, preferably on the anatomical structure, and/or location of fibrotic tissue in the vicinity of the target.

In some embodiments, the training of the machine-learning model is at least partially based on at least one variable feature. The variable feature may be selected from pulse date and time, pulse types, pulse power, pulse frequency, pulse duration, use of infiltration, use of anesthesia, combination with a therapy, treatment head position, and/or pressure exerted onto the tis-sue.

In some embodiments, the training of the machine-learning model is at least partially based on at least one monitoring feature. The monitoring feature may be selected from history of previous pulse parameters, number of pulses delivered on the current treatment slice, number of pulses delivered on the neighboring treatment slices, total energy delivered on the vein since the beginning of the treatment, pre and/or post-pulse images of the previous pulses, and/or extracted information (e.g. HEM area and/or HEM location with respect to the vein), and/or saved B-mode image collected during treatment.

In some embodiments, the anatomical structure comprises a vein, preferably a varicose vein.

In some embodiments, the pre-procedure and post-procedure digital data acquisition comprise a B-mode acquisition of at least a portion of the vein.

In some embodiments, the pre-procedure and post-procedure digital data comprise the diameter of the vein in several segments along the vein.

In some embodiments, the procedure comprises a HITU therapy of the vein.

In some embodiments, the outcome metric indicates the local effect of the HITU therapy on the vein, preferably a change in diameter of the vein before and after the procedure, for example by computing a difference or a ratio or a linear or nonlinear combination thereof.

In some embodiments, the pre-procedure and post-procedure digital data comprise the depth under skin of the vein, preferably in several segments along the vein.

In some embodiments, the procedure comprises at least one of a change in position of the patient, preferably from standing position to substantially lying position, positioning of a medical device, preferably a HITU treatment head, performing a local anesthesia or the injection of a fluid in the vicinity of the anatomical structure, for example, subcutaneously, or in a space delimited by one or several fascia.

The outcome metric may in particular reflect at least the change in the depth under skin of the anatomical structure.

The lying position may in particular be a Trendelenburg position.

In some embodiments, the procedure comprises daily activities following a HITU therapy of the vein.

In some embodiments, the outcome metric represents the evolution of the local effect of the HITU therapy on the vein. In particular, the outcome metric may represent a change of the diameter of the vein before and after the procedure, in particular a difference or a ratio or a linear or nonlinear combination thereof.

In some embodiments, the pre-procedure and post-procedure digital data acquisition comprises at least a B-mode acquisition captured substantially at the same position with respect to the anatomical structure.

The procedure may comprise delivering a HITU pulse.

The outcome metric may represent a change in the tissue properties of the anatomical structure such as a characteristic of hyperechoic mark, preferably one or a combination of the onset time, the area, the contour, the mask, the brightness of the HEM on the B-mode image, position and/or a dimension of the HEM.

Further disclosed is a computer-implemented method of determining an outcome metric associated with a procedure, in particular a High-Intensity Therapeutic Ultrasound (HITU) therapy. The method comprises obtaining digital data associated with an anatomical structure of a patient. The digital data comprises or is derived from data obtained in a medical imaging procedure. Using a machine-learning model, a value of the outcome metric is predicted based on the digital data. The machine-learning model may have been trained using the method of training a machine learning algorithm disclosed herein.

The computer-implemented methods disclosed herein may in particular include:

- estimating the chances of chances of success of a therapy, preferably a HITU therapy,
- estimating different success rates associated with different HITU therapy strategies (sets or ranges of values for variable features, including anesthesia and/or the combination with other treatment modalities,
- planning a therapy, preferably a HITU therapy, preferably by estimating appropriate values or ranges of variables parameters, preferably including treatment approaches not related to the medical device delivering the HITU therapy, such as anesthesia or combination with other treatment modalities,
- adapting a treatment planning during a treatment, for example in case of pain or patient movement,
- automating a HITU therapy by proposing values or ranges of values for variables features for a subsequent part of the procedure,
- using at least an actual or estimated depth under skin of the anatomical structure in treatment position as input for the prediction, in particular estimating the depth under the skin using a method disclosed herein.

The computer-implemented method disclosed herein may include estimating a probability of occurrence of a HEM, preferably based on at least one of a frequency of the HITU pulse and a power of the HITU pulse.

A computer program comprising instructions which, when the program is executed by a computer, cause the computer to carry out any of the methods disclosed herein.

## Claims

1. System (1) for gathering treatment data and/or generating a treatment plan for treating a patient with HITU, comprising

   - a computer device (10) which can be brought in operative connection with a memory (12);
   - wherein the computer device (10) is adapted to receive, via an interface (11), first imaging data of a body region and second imaging data (20) of the body region,
   - wherein the system (1) is preferably further adapted to create a first reconstructed model (22, 23, 23') of the body region before or without a treatment and a second reconstructed model (22, 23, 23") of the body region after or during treatment has been conducted based on the first and second imaging data (20), and/or preferably to receive a first reconstructed model (22, 23, 23') of the body region before a treatment and a second reconstructed model (22, 23, 23") of the body region after treatment forming first and second imaging data (20);
   - **characterized in that** the system (1), preferably the computer device (10), is further adapted to, based on the first and the second reconstructed models (22, 23, 23', 23''), generate an outcome metric for at least two segments (24', 24'') of the reconstructed first and second model (22, 23, 23', 23'') and save the outcome metric in the memory (12).

2. System (1) according to claim 1, further comprising at least one control unit (13, 13', 13") for controlling an imaging unit (15) and/or a treatment device (14).

3. System (1) according any of the preceding claims, wherein the computer device (10) is further adapted to receive and save treatment characteristics linked to the outcome metrics in the memory (12).

4. System (1) according to claim 2 and 3, further comprising a treatment device (14), preferably a treatment device (14) comprising HITU transducer.

5. System according to claim 4, wherein the control unit (13, 13', 13'') is adapted to record the treatment characteristics, and, via the interface (11), transmit the treatment characteristics to the computer device (10), preferably automatically.

**6.** System (1) according to any one of claims claim 2-4, further comprising an imaging device (15) which is controllable by the control unit (13, 13', 13"), particularly preferably a B-mode imaging device (15), wherein the computer device (10) is adapted to receive, via the interface (11), the first and second imaging data (20) from the imaging device (15).

**7.** System (1) according to claim 6, wherein the system comprises at least a first and a second imaging probe, preferably wherein the first imaging probe is an ultrasound hand probe.

**8.** System (1) according to claims 7 and 4, wherein the second imaging device is included in the treatment device.

**9.** System (1) according to any one of the preceding claims,
wherein the computer device (10) is adapted to register the first and second imaging data.

**10.** Method of gathering treatment data and/or evaluating treatment data, preferably using a system (1) according to any one of the preceding claims, comprising the steps of

- Obtaining first imaging data (20) of a body region representing a state before or without a treatment and second imaging data (20) of the body region representing a state after or during a treatment, and used treatment characteristics,
- Preferably saving said data on a memory device (12);
- Creating a first and a second model (22, 23, 23', 23"), preferably representing the body region before and after a treatment has been conducted;
- Preferably registering the first and second imaging data (20) and/or the first and second models (22, 23, 23', 23");
- Defining at least one outcome metric representing a change between the first and second model (22, 23, 23', 23"), particularly preferably a change in a diameter of a vessel (V),
- Determining said outcome metric for at least-two segments (24', 24") of the body region.

**11.** Method according to claim 10, also comprising a further step of collecting at least one treatment characteristic and corresponding position and/or orientation data.

**12.** Method according to any one of claims 10-11, wherein the outcome metric represents a change in a tissue characteristic before and after a treatment.

**13.** Method according to any one of claims 10-12, wherein the second imaging data (20) represents a state where the patient is in a position for therapy, and the outcome metric represents a change in a distance between a patient's skin (S) and a tissue (V) to be treated

**14.** Method according to any one of claims 10-13, further comprising the step of determining a correlation, preferably obtaining a model, between outcome metric and at least one of the treatment characteristics, preferably at least with a treatment parameter.

**15.** Method of defining at least one a treatment parameter for treating a patient, comprising the steps of

- Preferably obtaining imaging data (20) of a body region to be treated;
- Preferably selecting, from the imaging data (20), at least two segments (24', 24") of the body region;
- Automatically selecting a value of at least one treatment parameter which is suitable for achieving an optimum of an objective function, preferably wherein the optimization of the objective function is performed taking into account at least two segments.

**16.** Method of treating a patient, preferably with HITU, comprising the steps of:

- Preferably obtaining first imaging data (20), preferably a first model (22, 23, 23'), of a body region to be treated;
- optionally defining at least two segments (24', 24") from the imaging data (20) of the body region;
- Selecting a value of at least one treatment parameter which is suitable for achieving an optimization of an objective function, preferably wherein the optimization of the objective function is performed taking into account at least two segments, preferably using the method of claim 15;
- Treating the body region, preferably by impinging the body region with a HITU beam, using the selected value of the at least one treatment parameter.

17. Method according to claim 16, wherein the objective function is at least partially a function of a difference in the body region before and after the treatment, preferably for given values of fixed features.

18. Method according to any one of claims claim 16 or 17, further comprising a steps of

- obtaining second imaging data (20) after treating the body region and creating a second model;
- registering the first and second models (22, 23, 23', 23");
- Determining an achieved outcome metric;
- Storing the value of the at least one treatment characteristic, outcome metric, and preferably imaging data (20), in a memory (12).

19. Computer program product comprising instructions which, when the program is executed by a computer, cause the computer to carry out the steps of the method of any one of claims 10 to 18.

20. A training dataset for use in a method of training a machine-learning model, comprising first and second imaging data of a body region of a patient, wherein the first imaging data corresponds to a first state before a treatment and the second imaging data corresponds to a second state after the treatment has been conducted, wherein the first and second imaging data are registered.

21. A system for treating a patient, comprising a data processing apparatus having means for carrying out the method according to any one of claims 10-18, further comprising a memory on which a model and/or output, selected from a mathematical model and/or statistical model and/or an artificial intelligence model and/or an output of an inferential statistical analysis, is saved, wherein the model and/or output is obtained by training a machine-learning algorithm with a training dataset according to claim 20.

22. The system according to claim 21, wherein the model and/or output is adapted to output a treatment parameter based on imaging data and/or treatment characteristics input.

Fig. 1a

Fig. 1b

Fig. 1c

Fig. 1d

Fig. 1e

Fig. 2a

Fig. 2b

Fig. 2c

Fig. 3a

Fig. 3b

Fig. 3c

Fig. 3d

Fig. 4a

Fig. 4b

Fig. 4c

Fig. 4d

Fig. 5a

Fig. 5b

Fig. 6

Fig. 7a        Fig. 7b

Fig. 8

Fig. 9

Fig. 10

Fig. 11

Fig. 12

112

Heatmap

| | -1 | 0 | 1 | 2 | 3 |
|---|---|---|---|---|---|
| 2 | | | | | |
| 1 | | | 80 | | |
| 0 | | 150 | | | 100 |
| -1 | | | 120 | | |
| -2 | | | | | |

110

111

Fig. 13

Classification tree based on fix features

10000 slices
Success rate: 95 %

Pre-treatment diameter ≤ 14 mm      Pre-treatment diameter > 14 mm

9500 slices
Success rate: 96 %

500 slices
Success rate: 75 %

Classification tree based on variable features

Power ≤ 100 W    Power > 100 W

Number of pulses per treatment slice ≤ 3

Number of pulses per treatment slice > 3

375 slices
Success rate: 0 %

9125 slices
Success rate: 100 %

125 slices
Success rate: 0 %

375 slices
Success rate: 100 %

Fig. 14

V

127

128

126

| 5/5 pulses |
| 5/5 pulses |
| 2/3 pulses |
| 1/5 pulses |
| 0/4 pulses |
| 0/3 pulses |
| 0/2 pulses |
| 0/2 pulses |
| 0/2 pulses |
| 0/2 pulses |
| 0/2 pulses |
| 0/4 pulses |
| 0/4 pulses |
| 0/4 pulses |
| 0/3 pulses |
| 0/3 pulses |
| 0/3 pulses |
| 0/3 pulses |

123

Power selection

+20 W

300 W

-20 W

Pulse selection

0.5 s   1 s

Pulse

121

122

125

124

Fig. 15

Fig. 16

Fig. 17

Fig. 18

Fig. 19

EP 4 454 586 A1

Fig. 20

Fig. 21

49

Fig. 22

Fig. 23

211 212 213

Fig. 24

221 222 223 224 225 226 229 228

221 227

Fig. 25

Fig. 26

Fig. 27

Fig. 28

Fig. 29

Europäisches
Patentamt

European
Patent Office

Office européen
des brevets

# EUROPEAN SEARCH REPORT

Application Number

EP 23 31 5110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/157842 A1 (DAVIS CYNTHIA ELIZABETH LANDBERG [US] ET AL) 21 June 2012 (2012-06-21) * figures 1, 3 * * paragraphs [0029] – [0059] * ----- | 1-14,19 | INV. A61B34/10 A61B90/00 A61B34/00 A61N7/00 A61N7/02 |
| X | US 2007/010805 A1 (FEDEWA RUSSELL J [US] ET AL) 11 January 2007 (2007-01-11) * figures 1, 10-14B * * paragraphs [0042], [0043], [0058] – [0061], [0104] – [0108] * ----- | 1-6, 8-12,14, 19 | ADD. A61B34/20 |

TECHNICAL FIELDS
SEARCHED      (IPC)

A61B
A61N

The present search report has been drawn up for all claims

| Place of search | Date of completion of the search | Examiner |
|---|---|---|
| Munich | 30 November 2023 | Schleich, Florian |

EPO FORM 1503 03.82 (P04C01)

Europäisches
Patentamt

European
Patent Office

Office européen
des brevets

**Application Number**

**EP 23 31 5110**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

    see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

    1-14(completely); 19(partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**
**SHEET B**

Application Number

EP 23 31 5110

The Search Division considers that the present European patent application does not comply with the requirements of unity of invention and relates to several inventions or groups of inventions, namely:

1. claims: 1-14(completely); 19(partially)

    System adapted to, based on first and second reconstructed models, generate an outcome metric for at least two segments of a body region of the reconstructed first and second model and a corresponding method.
    ---

2. claims: 15-18(completely); 19(partially)

    Method of defining at least one treatment parameter for treating a patient comprising the step selecting a value of at least one treatment parameter which is suitable for achieving an optimum of an objective function.
    ---

3. claims: 20-22

    A training dataset for use in a method of training a machine-learning model and a corresponding system.
    ---

## ANNEX TO THE EUROPEAN SEARCH REPORT
## ON EUROPEAN PATENT APPLICATION NO.

EP 23 31 5110

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2023

| Patent document cited in search report | | Publication date | Patent family member(s) | | Publication date |
|---|---|---|---|---|---|
| US 2012157842 | A1 | 21-06-2012 | BR | PI1105359 A2 | 09-04-2013 |
| | | | CN | 102580260 A | 18-07-2012 |
| | | | US | 2012157842 A1 | 21-06-2012 |
| US 2007010805 | A1 | 11-01-2007 | BR | PI0614049 A2 | 09-03-2011 |
| | | | CN | 101222895 A | 16-07-2008 |
| | | | EP | 1901694 A2 | 26-03-2008 |
| | | | JP | 5064386 B2 | 31-10-2012 |
| | | | JP | 2009500126 A | 08-01-2009 |
| | | | US | 2007010805 A1 | 11-01-2007 |
| | | | US | 2008091123 A1 | 17-04-2008 |
| | | | US | 2008091124 A1 | 17-04-2008 |
| | | | US | 2009198131 A1 | 06-08-2009 |
| | | | US | 2015321027 A1 | 12-11-2015 |
| | | | US | 2016332005 A1 | 17-11-2016 |
| | | | WO | 2007008700 A2 | 18-01-2007 |

EPO FORM P0459

For more details about this annex : see Official Journal of the European Patent Office, No. 12/82

**REFERENCES CITED IN THE DESCRIPTION**

**Patent documents cited in the description**

- EP 1907065 A
- US 6968224 B
- US 8112292 B
- US 2020113543 A
- US 7693257 B
- US 2016174895 A
- DE 102014111066 A1
- EP 3720356 A